# EUROPEAN PATENT APPLICATION

(11) **EP 4 461 813 A2**
(43) Date of publication of application: **13.11.2024**
(21) Application number: 24189828.7
(22) Date of filing: 31.05.2018
(51) Int. Cl.: C12N 15/82

(54) **COMPOSITIONS AND METHODS FOR INCREASING SHELF-LIFE OF BANANA**

(30) Priority: 31.05.2017 GB 201708662
(62) Divisional of application: 18742593.9
(71) Applicant: Tropic Biosciences UK Limited, Colney, Norwich NR4 7GJ (GB)
(72) Inventor: MAORI, Eyal, Cambridge, CB4 3RQ (GB); GALANTY, Yaron, Cambridge, CB23 7PS (GB); PIGNOCCHI, Cristina, Norwich, NR9 3EP (GB); CHAPARRO GARCIA, Angela, Norwich, NR1 3RW (GB); MEIR, Ofir, Norwich, NR4 6PX (GB)
(74) Representative: J A Kemp LLP

(57) **Abstract**

A banana plant comprising a genome comprising a loss of function mutation in a nucleic acid sequence encoding a component in an ethylene biosynthesis pathway of the banana is provided. Also provides is a method of increasing shelf-life of banana.

## Description

### FIELD AND BACKGROUND OF THE INVENTION

The present invention, in some embodiments thereof, relates to compositions and methods for increasing shelf-life of banana.

Cultivated bananas and plantains are giant herbaceous plants within the genus *Musa.* They are both sterile and parthenocarpic so the fruit develops without seed. The cultivated hybrids and species are mostly triploid (2*n* = *3x* = 33; a few are diploid or tetraploid), and most have been propagated from mutants found in the wild.

Bananas are one of the top ten world food crops. Bananas are eaten both raw and cooked, depending on the cultivar. About 60 % of bananas are eaten raw, as a dessert fruit, and the other 40 % are cooked during processes steaming, boiling, roasting, and frying. More than 120 million tonnes of banana fruit are produced each year, with the three biggest producers, India, Uganda, and China, consuming almost all of what they produce domestically.

Banana belongs to a climacteric fruit, after harvesting, green banana has to undergo climacteric change through its ripening process, including production of internal ethylene, hydrolysis of starch and protopectin, and the like, till fruit flesh softened, sweetness increased, and fragrance produced, and then, its dietary value can be increased.

Conventionally, banana is harvested in advance, and its transportation and storage period is prolonged by the ripening progress. However, banana fruit may often undergo ripening due to the production of ethylene during the transportation process. Furthermore, the fruit may be over-ripened and become spoiled, lowering the marker value significantly. Accordingly, control on the biosynthesis of ethylene can be used to provide a method to control ripening of banana.

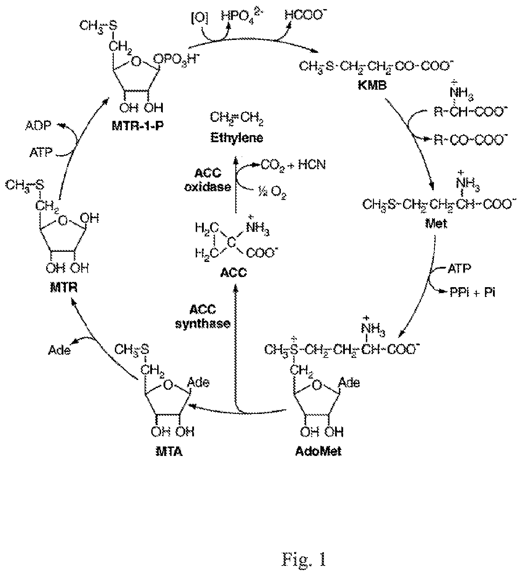

Ethylene is a plant hormone present in gaseous form, which can affect a number of physiological and biochemical reactions in plant. Ethylene plays an important role in the growth, development, and stress-response of plant, for example, when a plant is subjected to flooding, mechanical injury, bacterial infection, aging of leaf and flower, fruit ripening, and the like, it will produce ethylene. The biosynthesis pathway of ethylene comprises of conversion of methionine into S-Adenosyl-methionine (AdoMet) with the aid of AdoMet synthase, synthesis of 1-aminocyclopropane-1-carboxylic acid (ACC) from AdoMet with the aid of ACC synthase (ACS), and then oxidation of ACC into ethylene with the aid of ACC oxidase (ACO) (see Figure 1, adapted from Rudus et al. 2013, Volume 35, Issue 2, pp 295-307). It is known that ACO is the last enzyme used in the biosynthesis pathway of ethylene, and as a result, inhibition on ACO gene or protein expression thereof can inhibit/knock-down the biosynthesis of ethylene, and further, to achieve the object of retarding the after-ripening of a fruit.

Unlike most other major food crops, bananas are difficult to genetically improve. The challenge is that nearly all banana cultivars and landraces are triploids, with high levels of male and female infertility. There are a number of international conventional breeding programs and many of these are developing new cultivars. However, it is virtually impossible to backcross bananas, thus excluding the possibility of introgressing new traits into a current cultivar.

Thus, to meet the challenge of increasing global demand for food production, the typical approaches to improving agricultural productivity (e.g. enhanced yield or engineered pest resistance) have relied on either mutation breeding or introduction of novel genes into the genomes of crop species by transformation. These processes are inherently nonspecific and relatively inefficient. For example, plant transformation methods deliver exogenous DNA that integrates into the genome at random locations. Thus, in order to identify and isolate transgenic plant lines with desirable attributes, it is necessary to generate hundreds of unique random integration events per construct and subsequently screen for the desired individuals. As a result, conventional plant trait engineering is a laborious, time-consuming, and unpredictable undertaking. Furthermore, the random nature of these integrations makes it difficult to predict whether pleiotropic effects due to unintended genome disruption have occurred.

The random nature of the current transformation processes requires the generation of hundreds of events for the identification and selection of transgene event candidates (transformation and event screening is rate limiting relative to gene candidates identified from functional genomic studies). In addition, depending upon the location of integration within the genome, a gene expression cassette may be expressed at different levels as a result of the genomic position effect. As a result, the generation, isolation and characterization of plant lines with engineered genes or traits has been an extremely labor and cost-intensive process with a low probability of success. In addition to the hurdles associated with selection of transgenic events, some major concerns related to gene confinement and the degree of stringency required for release of a transgenic plants into the environment for commercial applications arise.

Recent advances in genome editing techniques have made it possible to alter DNA sequences in living cells. Genome editing is more precise than conventional crop breeding methods or standard genetic engineering (transgenic or GM) methods. By editing only a few of the billions of nucleotides (the building blocks of genes) in the cells of plants, these new techniques might be the most effective way to get crops to grow better in harsh climates, resist pests or improve nutrition. Because the more precise the technique, the less of the genetic material is altered, so the lower the uncertainty about other effects on how the plant behaves.

The most established method of plant genetic engineering using CRISPR Cas9 genome editing technology requires the insertion of new DNA into the host's genome. This insert, transfer DNA (T-DNA), carries several transcriptional units in order to achieve successful CRISPR Cas9 genome edits. These commonly consist of an antibiotic resistance gene to select for transgenic plants, the Cas9 machinery, and several sgRNA units. Because of the integration of foreign DNA into the genome, plants generated this way are classified as transgenic or genetically modified (GM). Once a genome edit has been established in the host, this T-DNA backbone can be removed through sexual propagation and breeding, as the CRISPR Cas9 machinery is no longer needed to maintain the phenotype. However, as mentioned, banana species are parthenocarpic (do not produce viable seeds) rendering the removal of T-DNA backbone by sexual reproduction impossible.

Additional background art includes:
U.S. Appl, Publ. No. 20130097732
U.S. Patent Application 20140075593;
Zhang, Y., et al., Efficient and transgene-free genome editing in wheat through transient expression of CRISPR/Cas9 DNA or RNA. Nat Commun, 2016. 7: p. 12617;
Woo, J.W., et al., DNA-free genome editing in plants with preassembled CRISPR-Cas9 ribonucleoproteins. Nat Biotechnol, 2015. 33(11): p. 1162-4;
Svitashev, S., et al., Genome editing in maize directed by CRISPR-Cas9 ribonucleoprotein complexes. Nat Commun, 2016. 7: p. 13274;
Luo, S., et al., Non-transgenic Plant Genome Editing Using Purified Sequence-Specific Nucleases. Mol Plant, 2015. 8(9): p. 1425-7.
Hoffmann 2017 PlosOne 12(2):e0172630;
Chiang et al., 2016. SP1,2,3. Sci Rep. 2016 Apr 15;6:24356.

### SUMMARY OF THE INVENTION

According to an aspect of some embodiments of the present invention there is provided a banana plant comprising a genome comprising a loss of function mutation in a nucleic acid sequence encoding a component in an ethylene biosynthesis pathway of the banana.

According to an aspect of some embodiments of the present invention there is provided a method of increasing shelf-life of banana, the method comprising:
(a) subjecting a banana plant cell to a DNA editing agent directed at a nucleic acid sequence encoding a component in an ethylene biosynthesis pathway of the banana to result in a loss of function mutation in the nucleic acid sequence encoding the ethylene biosynthesis pathway and
(b) regenerating a plant from the plant cell.

According to some embodiments of the invention, the method further comprises harvesting fruit from the plant.

According to some embodiments of the invention, the plant is devoid of a transgene encoding the DNA editing agent.

According to some embodiments of the invention, the mutation is in a homozygous form.

According to some embodiments of the invention, the plant or ancestor thereof having been treated with a DNA editing agent directed to the genomic sequence encoding the component in the ethylene biosynthesis pathway.

According to some embodiments of the invention, the mutation is selected from the group consisting of a deletion, an insertion an insertion/deletion (Indel) and a substitution.

According to some embodiments of the invention, the component in the ethylene biosynthesis pathway is selected from the group consisting of 1-aminocyclopropane-1-carboxylate synthase (ACS) and ACC oxidase (ACO)

According to an aspect of some embodiments of the present invention there is provided a nucleic acid construct comprising a nucleic acid sequence encoding a DNA editing agent directed at a nucleic acid sequence encoding a component in an ethylene biosynthesis pathway of a banana being operably linked to a plant promoter.

According to some embodiments of the invention, the DNA editing agent is of a DNA editing system selected from the group consisting of selected from the group consisting of meganucleases, Zinc finger nucleases (ZFNs), transcription-activator like effector nucleases (TALENs) and CRISPR-Cas.

According to some embodiments of the invention, the DNA editing agent is of a DNA editing system comprising CRISPR-Cas.

According to some embodiments of the invention, the component in the ethylene biosynthesis pathway is selected from the group consisting of Ma04_g35640 (SEQ ID NO: 9) and Ma07_g19730 (SEQ ID NO: 27).

According to some embodiments of the invention, the component in the ethylene biosynthesis pathway is selected from the group consisting of Ma09_g19150 (SEQ ID NO: 13), Ma04_g35640 (SEQ ID NO: 9), Ma04_g31490 (SEQ ID NO: 8), Ma01_g11540 (SEQ ID NO: 20) and Ma07_g19730 (SEQ ID NO: 27).

According to some embodiments of the invention, the component in the ethylene biosynthesis pathway is selected from the group consisting of Ma04_g35640 (SEQ ID NO: 9) and Ma07_g19730 (SEQ ID NO: 27).

According to some embodiments of the invention, the component in the ethylene biosynthesis pathway is selected from the group consisting of Ma09_g19150 (SEQ ID NO: 13), Ma04_g31490 (SEQ ID NO: 8) and Ma01_g11540 (SEQ ID NO: 20).

According to some embodiments of the invention, the DNA editing agent is directed at nucleic acid coordinates which specifically target more than one nucleic acid sequence encoding the component in the ethylene biosynthesis pathway.

According to some embodiments of the invention, the DNA editing agent comprises a nucleic acid sequence at least 99 % identical to a nucleic acid sequence selected from the group consisting of SEQ ID NO: 47-54.

According to some embodiments of the invention, the DNA editing agent comprises a nucleic acid sequence at least 99 % identical to a nucleic acid sequence set forth in SEQ ID NO: 47.

According to some embodiments of the invention, the DNA editing agent comprises a nucleic acid set forth in SEQ ID NO: 47.

According to some embodiments of the invention, the DNA editing agent comprises a plurality of nucleic acid sequences set forth in SEQ ID NO: 47-54.

According to some embodiments of the invention, the DNA editing agent comprises a plurality of nucleic acid sequences set forth in SEQ ID NO: 47, 49 or 50.

According to some embodiments of the invention, the DNA editing agent comprises a plurality of nucleic acid sequences set forth in SEQ ID NO: 51 and 53.
According to some embodiments of the invention, the banana plant is non-transgenic.

According to an aspect of some embodiments of the present invention there is provided a plant part of the plant as described herein.

According to some embodiments of the invention, the plant part is a fruit.

According to some embodiments of the invention, the fruit is dry.

According to an aspect of some embodiments of the present invention there is provided a method of producing banana, the method comprising:
(a) growing the plant as described herein; and
(b) harvesting fruit from the plant.

According to an aspect of some embodiments of the present invention there is provided a processed banana product comprising genomic banana DNA comprising a loss of function mutation in a nucleic acid sequence encoding a component in an ethylene biosynthesis pathway of the banana.

According to an aspect of some embodiments of the present invention there is provided a banana plant, or part thereof, comprising a loss of function mutation introduced into a genomic nucleic acid sequence encoding a protein that is a component in an ethylene biosynthesis pathway of the banana, wherein the mutation results in a reduced level or reduced activity of the protein as compared to a banana plant lacking the loss of function mutation.

According to some embodiments of the invention, the plant comprises one or more non-natural loss of function mutations introduced into one or more genomic nucleic acid sequences encoding one or more proteins that are components in an ethylene biosynthesis pathway of the banana, wherein the one or more mutations each results in reduced levels or reduced activities of the protein as compared to a banana plant lacking the loss of function mutation.

According to some embodiments of the invention, the one or more proteins are selected from the group consisting of 1-aminocyclopropane-1-carboxylate synthase (ACS) and ACC oxidase (ACO).

According to some embodiments of the invention, the ACS protein genomic nucleic acid sequence comprises a nucleic acid sequence at least 85% identical to, at least 90% identical to, at least 95% identical to, or is a nucleic acid sequence selected from the group consisting of Ma01_g07800.1 (SEQ ID NO: 1), Ma01_g12130.1 (SEQ ID NO: 2), Ma02_g10500.1 (SEQ ID NO: 3), Ma03_g12030.1 (SEQ ID NO: 4), Ma03_g27050.1 (SEQ ID NO: 5), Ma04_g01260.1 (SEQ ID NO: 6), Ma04_g24230.1 (SEQ ID NO: 7), Ma04_g31490.1 (SEQ ID NO: 8), Ma04_g35640.1 (SEQ ID NO: 9), Ma04_g37400.1 (SEQ ID NO: 10), Ma05_g08580.1 (SEQ ID NO: 11), Ma05_g13700.1 (SEQ ID NO: 12), Ma09_g19150.1 (SEQ ID NO: 13), and Ma10_g27510.1 (SEQ ID NO: 14); and wherein the ACO protein genomic nucleic acid sequence comprises a nucleic acid sequence at least 85% identical to, at least 90% identical to, at least 95% identical to, or is a nucleic acid sequence selected from the group consisting of Ma09_g04370.1 (SEQ ID NO: 15), Ma06_g17160.1 (SEQ ID NO: 16), Ma11_g05490.1 (SEQ ID NO: 17), Ma00_g04490.1 (SEQ ID NO: 18), Ma07_g15430.1 (SEQ ID NO: 19), Ma01_g11540.1 (SEQ ID NO: 20), Ma10_g16100.1 (SEQ ID NO: 21), Ma05_g08170.1 (SEQ ID NO: 22), Ma06_g14430.1 (SEQ ID NO: 23), Ma05_g09360.1 (SEQ ID NO: 24), Ma11_g22170.1 (SEQ ID NO: 25), Ma05_g31690.1 (SEQ ID NO: 26), Ma07_g19730.1 (SEQ ID NO: 27), Ma06_g02600.1 (SEQ ID NO: 28), Ma10_g05270.1 (SEQ ID NO: 29), Ma06_g14370.1 (SEQ ID NO: 30), Ma11_g05480.1 (SEQ ID NO: 31), Ma06_g14410.1 (SEQ ID NO: 32), Ma06_g14420.1 (SEQ ID NO: 33), Ma06_g34590.1 (SEQ ID NO: 34), Ma02_g21040.1 (SEQ ID NO: 35), Ma11_g04210.1 (SEQ ID NO: 36), Ma05_g12600.1 (SEQ ID NO: 37), Ma04_g23390.2 (SEQ ID NO: 38), Ma03_g06970.1 (SEQ ID NO: 39), Ma05_g09980.1 (SEQ ID NO: 40), Ma04_g36640.1 (SEQ ID NO: 41), Ma11_g04180.1 (SEQ ID NO: 42), Ma11_g02650.1 (SEQ ID NO: 43), and Ma00_g04770.1 (SEQ ID NO: 44).
According to some embodiments of the invention, the genomic nucleic acid sequence encoding the protein component in the ethylene biosynthesis pathway comprises a nucleic acid sequence at least 85% identical to, at least 90% identical to, at least 95% identical to, or is a nucleic acid sequence selected from the group consisting of Ma09_g19150 (SEQ ID NO: 13), Ma04_g35640 (SEQ ID NO: 9), Ma04_g31490 (SEQ ID NO: 8), Ma01_g11540 (SEQ ID NO: 20) and Ma07_g19730 (SEQ ID NO: 27).

According to some embodiments of the invention, the genomic nucleic acid sequence encoding the protein component in the ethylene biosynthesis pathway comprises a nucleic acid sequence at least 85% identical to, at least 90% identical to, at least 95% identical to, or is a nucleic acid sequence selected from the group consisting of Ma04_g35640 (SEQ ID NO: 9), and Ma07_g19730 (SEQ ID NO: 27).

According to some embodiments of the invention, the genomic nucleic acid sequence encoding the protein component in the ethylene biosynthesis pathway comprises a nucleic acid sequence at least 85% identical to, at least 90% identical to, at least 95% identical to, or is a nucleic acid sequence selected from the group consisting of Ma09_g19150 (SEQ ID NO: 13), Ma04_g31490 (SEQ ID NO: 8), and Ma01_g11540 (SEQ ID NO: 20).

According to some embodiments of the invention, the non-natural loss of function mutation was introduced using a DNA editing agent.

According to some embodiments of the invention, the plant does not comprise a transgene encoding the DNA editing agent, a transgene encoding a selectable marker or a reporter, or does not comprising a transgene encoding any of the DNA editing agent, the selectable marker, or the reporter.

According to some embodiments of the invention, the DNA editing agent comprised a DNA editing system selected from the group consisting of meganucleases, Zinc finger nucleases (ZFNs), transcription-activator like effector nucleases (TALENs) and CRISPR-Cas.

According to some embodiments of the invention, the DNA editing agent was CRISPR-Cas.

According to some embodiments of the invention, the mutation is homozygous.

According to some embodiments of the invention, the mutation is selected from the group consisting of a deletion, an insertion, an insertion/deletion (Indel), and a substitution.

According to an aspect of some embodiments of the present invention there is provided a nucleic acid construct comprising a nucleic acid sequence encoding a DNA editing agent and a DNA targeting agent, wherein the targeting agent targets the editing agent to a genomic nucleic acid sequence encoding a protein component in an ethylene biosynthesis pathway of a banana to introduce a loss of function mutation in to the genomic nucleic acid sequence, wherein the editing and targeting agents are operably linked to a plant promoter and wherein the mutation results in a reduced level or reduced activity of the protein as compared to a banana plant lacking the loss of function mutation.

According to some embodiments of the invention, the DNA editing agent and the DNA targeting agent generate one of the mutations in the genome of the plant of any one of claims 1-13.

According to some embodiments of the invention, the DNA targeting agent is designed to target nucleic acids which are common to more than one genomic nucleic acid sequence encoding a component in the ethylene biosynthesis pathway.

According to some embodiments of the invention, the DNA targeting agent comprises a nucleic acid sequence at least 99 % identical to a nucleic acid sequence selected from the group consisting of SEQ ID NO: 47-54.

According to some embodiments of the invention, the DNA editing agent comprises a nucleic acid sequence at least 99 % identical to a nucleic acid sequence set forth in SEQ ID NO: 47.

According to some embodiments of the invention, the DNA editing agent comprises a nucleic acid set forth in SEQ ID NO: 47.

According to some embodiments of the invention, the nucleic acid construct comprises two or more DNA editing agent comprises selected from the nucleic acid sequences set forth in SEQ ID NO: 47-54.

According to some embodiments of the invention, the nucleic acid construct comprises two or more DNA editing agent comprises selected from the nucleic acid sequences set forth in SEQ ID NO: 47, 49 or 50.

According to some embodiments of the invention, the nucleic acid construct comprises at least two DNA editing agent comprising the nucleic acid sequences set forth in SEQ ID NO: 51 and 53.

According to an aspect of some embodiments of the present invention there is provided a method of increasing shelf-life of banana, the method comprising:
(a) transforming one or more cells of a banana plant with the nucleic acid construct of any one of claims 14-22;
(b) generating the loss of function mutation in the genomic nucleic acid sequence encoding the protein component of the ethylene biosynthesis pathway, wherein the mutation results in the reduced level or reduced activity of the protein; and
(c) regenerating a plant from the plant cell.
According to some embodiments of the invention, the DNA editing agent is CRISPR-Cas and the DNA targeting agent is an sgRNA.

According to some embodiments of the invention, the genomic nucleic acid sequence encoding a protein component in an ethylene biosynthesis pathway of the banana is selected from the group consisting of Ma09_g19150 (SEQ ID NO: 13), Ma04_g35640 (SEQ ID NO: 9), Ma04_g31490 (SEQ ID NO: 8), Ma01_g11540 (SEQ ID NO: 20) and Ma07_g19730 (SEQ ID NO: 27).

According to some embodiments of the invention, the sgRNA DNA targeting agent is selected from the group consisting of sg-183 (SEQ ID NO: 47), sg-184 (SEQ ID NO: 48), sg-188 (SEQ ID NO: 49), sg-189 (SEQ ID NO: 50), sg-190 (SEQ ID NO: 51), sg-191 (SEQ ID NO: 52), sg-194 (SEQ ID NO: 53), and sg-195 (SEQ ID NO: 54).

According to some embodiments of the invention, the loss of function mutation is as described herein.

According to an aspect of some embodiments of the present invention there is provided a mutant banana plant comprising mutant bananas wherein the mutant plant comprises a mutation in a gene encoding an 1-aminocyclopropane-1-carboxylate synthase (ACS) protein wherein the activity of the ACS protein in the mutant banana plant is reduced compared to the activity of the protein from a banana plant lacking the mutation and wherein the mutant banana fruit ripen slower than bananas from a banana plant lacking the mutation.

According to an aspect of some embodiments of the present invention there is provided a mutant banana plant comprising mutant bananas wherein the mutant plant comprises a mutation in gene Ma09_g19150 (SEQ ID NO: 13) wherein gene Ma09_g19150 encodes protein 1-aminocyclopropane-1-carboxylate synthase (ACS) wherein the activity of protein ACS in the mutant banana plant is reduced compared to the activity of the protein from a banana plant lacking the mutation and wherein the mutant banana fruit ripen slower than bananas from a banana plant lacking the mutation.

According to an aspect of some embodiments of the present invention there is provided a mutant banana plant comprising mutant bananas wherein the mutant plant comprises a mutation in gene Ma04_g35640 (SEQ ID NO: 9) wherein gene Ma04_g35640 encodes protein 1-aminocyclopropane-1-carboxylate synthase (ACS) wherein the activity of protein ACS in the mutant banana plant is reduced compared to the activity of the protein from a banana plant lacking the mutation and wherein the mutant banana fruit ripen slower than bananas from a banana plant lacking the mutation.

According to an aspect of some embodiments of the present invention there is provided a mutant banana plant comprising mutant bananas wherein the mutant plant comprises a mutation in gene Ma04_g31490 (SEQ ID NO: 8) wherein gene Ma04_g31490 encodes protein 1-aminocyclopropane-1-carboxylate synthase (ACS) wherein the activity of protein ACS in the mutant banana plant is reduced compared to the activity of the protein from a banana plant lacking the mutation and wherein the mutant banana fruit ripen slower than bananas from a banana plant lacking the mutation.

According to an aspect of some embodiments of the present invention there is provided a mutant banana plant comprising mutant bananas wherein the mutant plant comprises a mutation in a gene encoding an ACC oxidase (ACO) protein wherein the activity of the ACO protein in the mutant banana plant is reduced compared to the activity of the protein from a banana plant lacking the mutation and wherein the mutant banana fruit ripen slower than bananas from a banana plant lacking the mutation.

According to an aspect of some embodiments of the present invention there is provided a mutant banana plant comprising mutant bananas wherein the mutant plant comprises a mutation in gene Ma01_g11540 (SEQ ID NO: 20) wherein gene Ma01_g11540 encodes protein ACC oxidase (ACO) wherein the activity of protein ACO in the mutant banana plant is reduced compared to the activity of the protein from a banana plant lacking the mutation and wherein the mutant banana fruit ripen slower than bananas from a banana plant lacking the mutation.

According to an aspect of some embodiments of the present invention there is provided a mutant banana plant comprising mutant bananas wherein the mutant plant comprises a mutation in gene Ma07_g19730 (SEQ ID NO: 27) wherein gene Ma07_g19730 encodes protein ACC oxidase (ACO) wherein the activity of protein ACO in the mutant banana plant is reduced compared to the activity of the protein from a banana plant lacking the mutation wherein the mutant bananas ripen slower than bananas from a banana plant lacking the mutation.

According to an aspect of some embodiments of the present invention there is provided a method of producing banana, the method comprising:
(a) growing the plant as described herein; and
(b) harvesting fruit from the plant.

According to some embodiments of the invention, the plant, or part thereof, is a plant part.

According to some embodiments of the invention, the plant part is a fruit.

According to an aspect of some embodiments of the present invention there is provided a processed banana product comprising the plant part.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWING(S)

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Fig. 1 is a scheme of the ethylene biosynthesis pathway taken from Bleecker and Kende. 2000. Annu. Rev. Cell. Dev 16: 1-18.

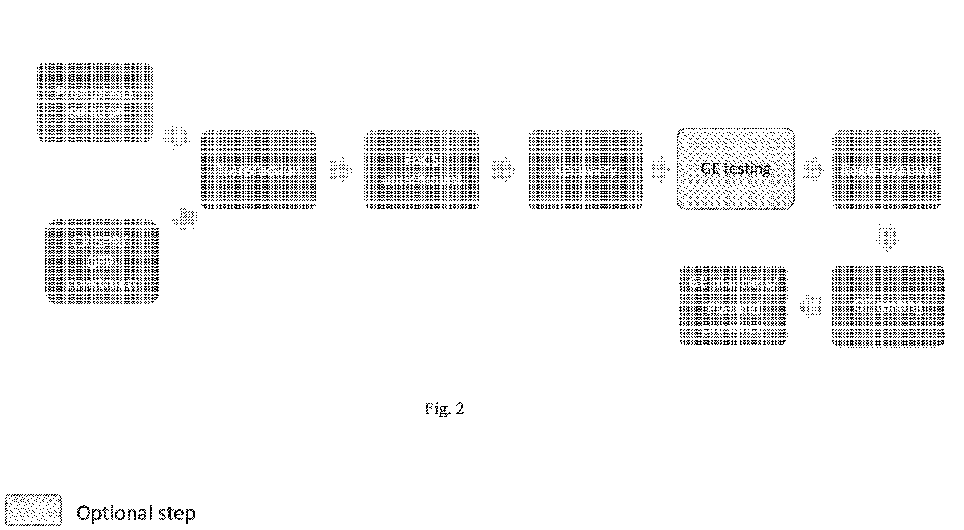

Fig. 2 is a flowchart of an embodiment of the method of selecting cells comprising a genome editing event;

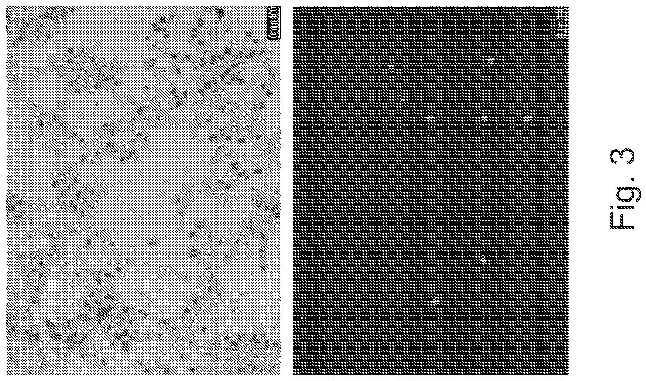

Fig. 3 shows positive transfection of banana protoplasts with mCherry plasmids. 1×10⁶ banana protoplasts were transfected using PEG with plasmid pAC2010 carrying mCherry (fluorescent marker). 3 days post-transfection, the transfection efficiency was analysed under a fluorescent microscope. The figure shows banana protoplasts, upper panel brightfield, lower panel fluorescence.

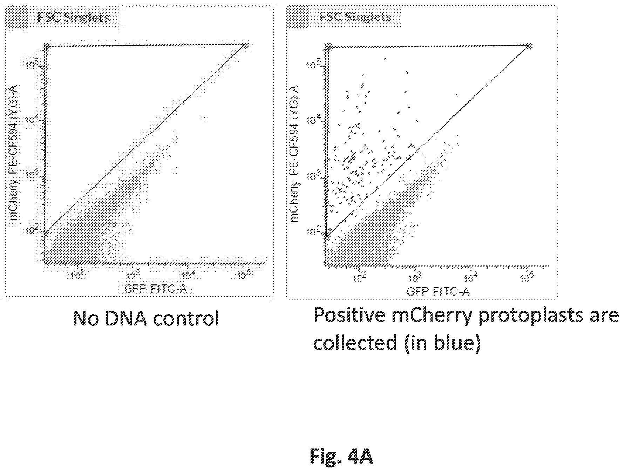

Fig. 4A shows FACS enrichment of positive mCherry banana. 1×10⁶ banana protoplasts were transfected using PEG with plasmid pAC2010 carrying the fluorescent marker mCherry. Three days post-transfection protoplasts were analyzed by FACS, all mCherry-positive cells were sorted and collected.

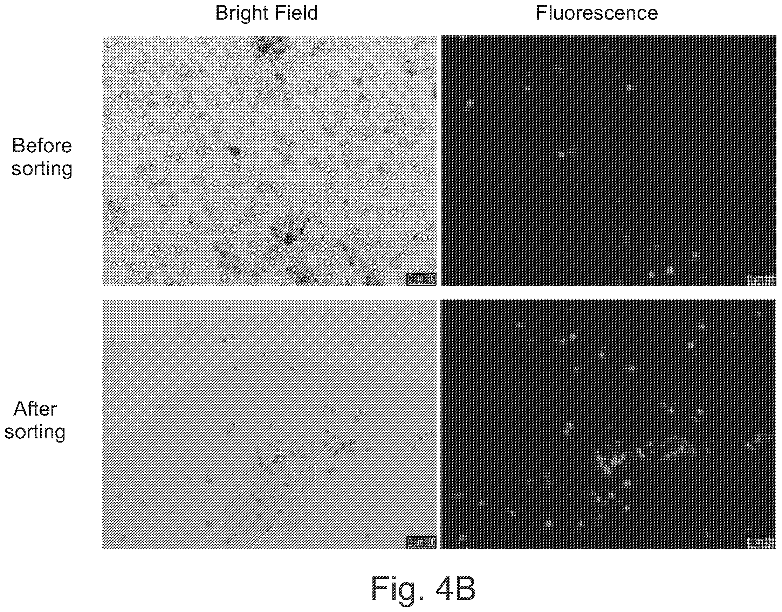

Fig. 4B shows FACS enrichment of positive mCherry banana protoplasts. Enrichment of mCherry banana protoplasts was confirmed by fluorescent microscopy. Unsorted (upper panels) and sorted (lower panels) transfected protoplasts were imaged with a fluorescent microscope at 3days post transfection.

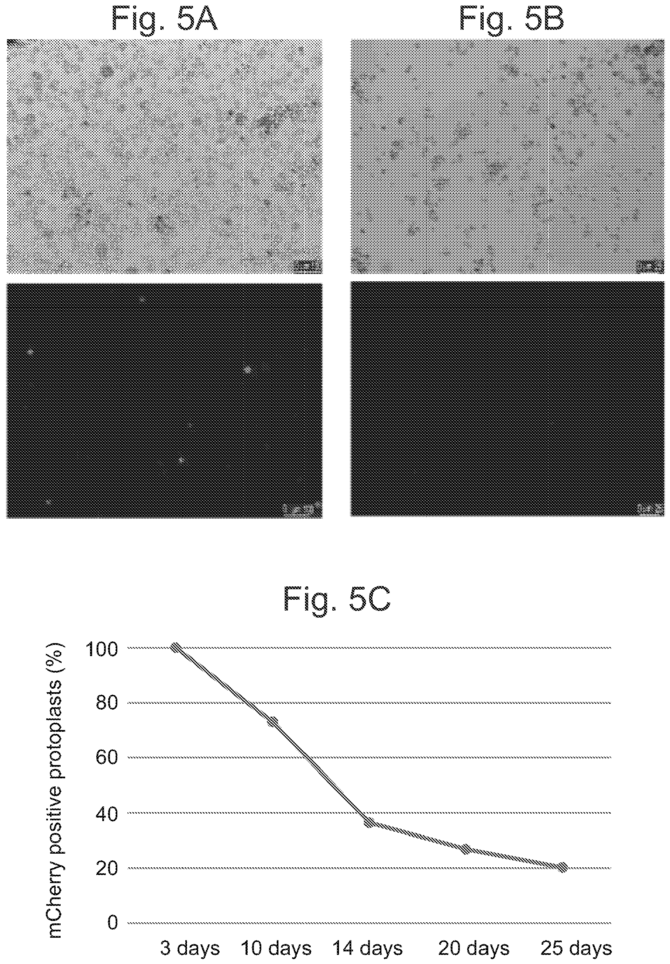

Figs. 5A-C show the decrease of mCherry positive banana protoplasts over time indicating transient transformation events. Banana protoplasts transfected with a plasmid carrying the mCherry fluorescent marker were imaged at 3 (Figure 5A) and 10 (Figure 5B) days post transfection. Figure 5C. Progressive reduction in number of mCherry positive protoplasts up to 25 days post transfection was observed as measured by FACS. 100 % represents the proportion of cherry-expressing cells at 3 days post-transfection.

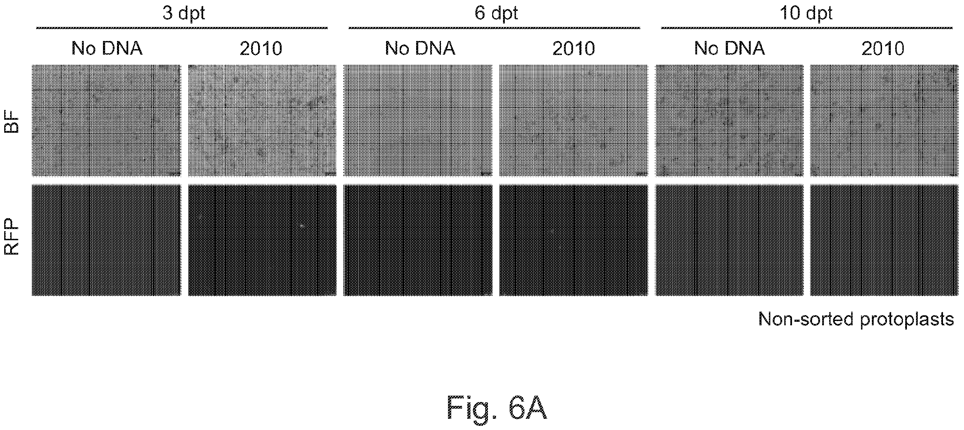

Fig. 6A shows the decrease of mCherry-positive banana protoplasts over time indicating transient transformation events on non-sorted protoplasts and imaged before FACS. Musa acuminata protoplasts were transfected with a plasmid carrying the mCherry fluorescent marker (pAC2010) or with no DNA. Non-sorted protoplasts were imaged at 3, 6, and 10 days post transfection as indicated. Microscopy images show the progressive reduction in number and intensity of mCherry-positive protoplasts along time. BF (Bright field).

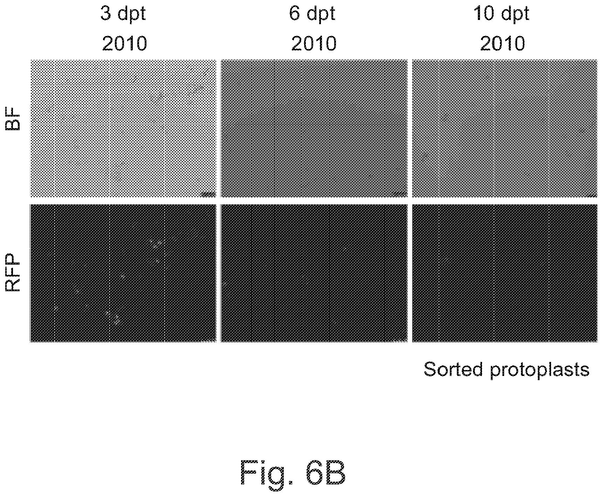

Fig. 6B shows the decrease of mCherry-positive protoplasts over time indicating transient transformation events on sorted protoplasts and imaged after **FACS.** *Musa acuminata* protoplasts transfected with a plasmid carrying the mCherry fluorescent marker (2010) were sorted and imaged at 3, 6, and 10 days post transfection as indicated. Microscopy images show the progressive reduction in number and intensity of mCherry-positive protoplasts along time. BF (Bright field).

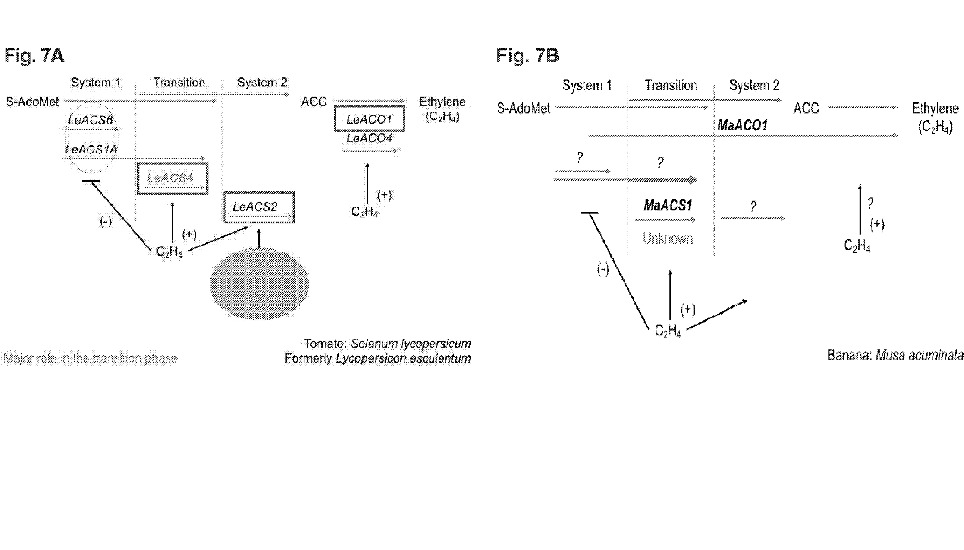

Figs. 7A-B is a schematic illustration of the ethylene biosynthesis and regulation during the system 1 to system 2 transition in *S. lycopersicum* and *M. acuminata.* Simplified scheme of the ethylene two-step biochemical pathway from S-adenosyl-L-methionine (S-Ado-Met) to 1-aminocyclopropane-1-carboxylic acid (ACC) to ethylene and the genes involved in the transition from system 1 to system 2 during tomato (Figure 7A) and banana fruit ripening (Figure 7B). The transition from system 1 to system 2 depends on gene expression regulation of several members of the ACC synthase (ACS) and ACC oxidase (ACO) gene families. Purple boxes indicate the tomato genes that were selected for further analysis. Tomato scheme was adapted from Alexander and Grierson, 2002. Journal of Experimental Botany, Vol. 53, No. 377, pp. 2039-2055; Cara and Giovannoni, 2008. Plant Science Vol. 175, pp. 106-113; and Pech et al., 2012. Annual Plant Reviews, Vol. 44, pp. 275-304. Banana scheme was based on the tomato findings and Liu et al., 1999. Plant Physiology, Vol 121, pp. 1257-1265 and Rudus et al., 2013. Acta Physiol Plant. Vol 35, pp. 295-307.

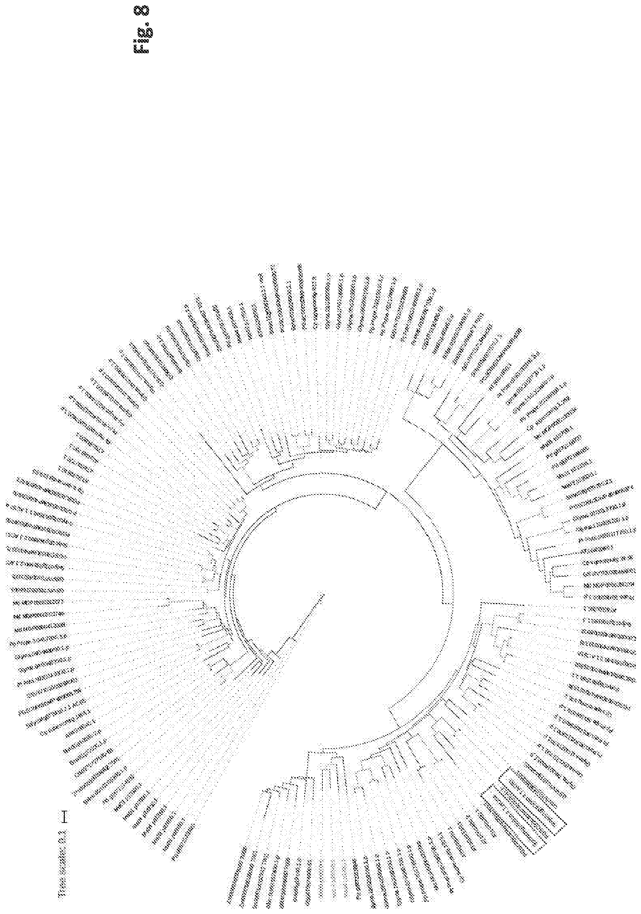

Fig. 8 is a schematic illustration of the evolutionary relationships of ACC synthase (ACS) genes. The evolutionary history was inferred using the Neighbor-Joining method. The percentage of replicate trees in which the associated taxa clustered together in the bootstrap test (1000 replicates) are shown as colored branches (red < 20%; blue 50%; green > 90%). Dashed purple boxes indicate the tomato genes that have been shown to be involved during tomato fruit ripening and that were used as query sequences to retrieve closely-related genes in the genome of *M. acuminata.* Gene IDs in orange indicate *M. acuminata* candidate genes that are the most likely closest homologs to the characterized tomato genes involved in fruit ripening.

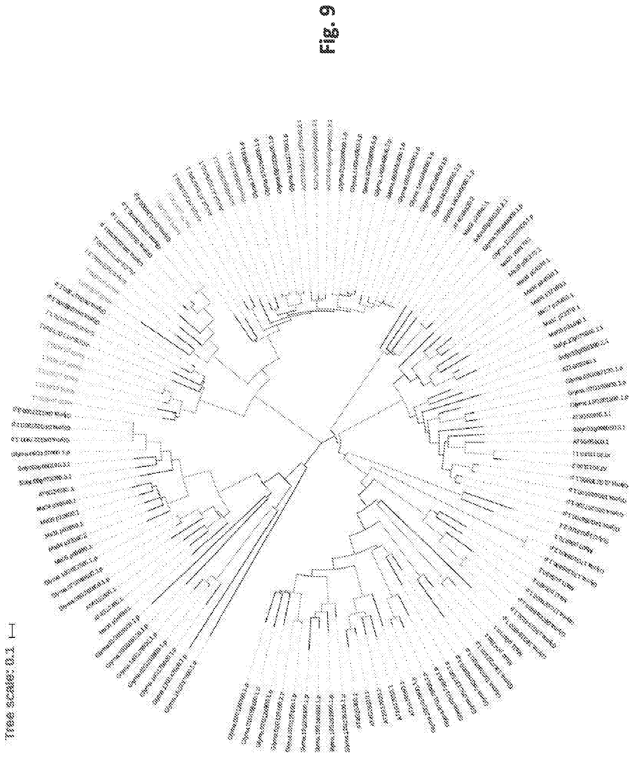

Fig. 9 is a schematic illustration of the evolutionary relationships of ACC oxidase (ACO) genes. The evolutionary history was inferred using the Neighbor-Joining method. The percentage of replicate trees in which the associated taxa clustered together in the bootstrap test (1000 replicates) are shown as colored branches (red < 20%; blue 50%; green > 90%). Gene IDs in purple or red indicate the genes from Arabidopsis or tomato, respectively, that have been characterized during fruit ripening and that were used as query sequences to retrieve closely-related genes in the genome of *M. acuminata.* Gene IDs in orange indicate *M. acuminata* candidate genes that are the most likely closest homologs (to tomato and Arabidopsis) to the characterized tomato genes involved in fruit ripening.

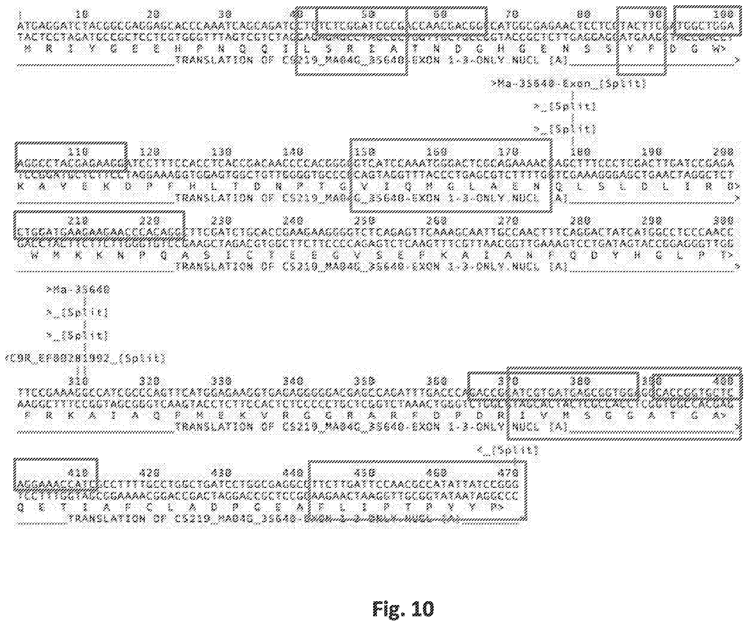

Fig. 10 shows an example of sgRNAs selection. After using publicly available algorithms to find and design sgRNAs in the sequence of interest, a manual curation step ensures the selection of sgRNAs that overlap with regions that have been shown empirically or predicted to be important for protein function (red boxes). Blue boxes highlight the positions where sgRNAs were designed. According to embodiments of the invention, sgRNAs are selected overlapping the blue and the red boxes.

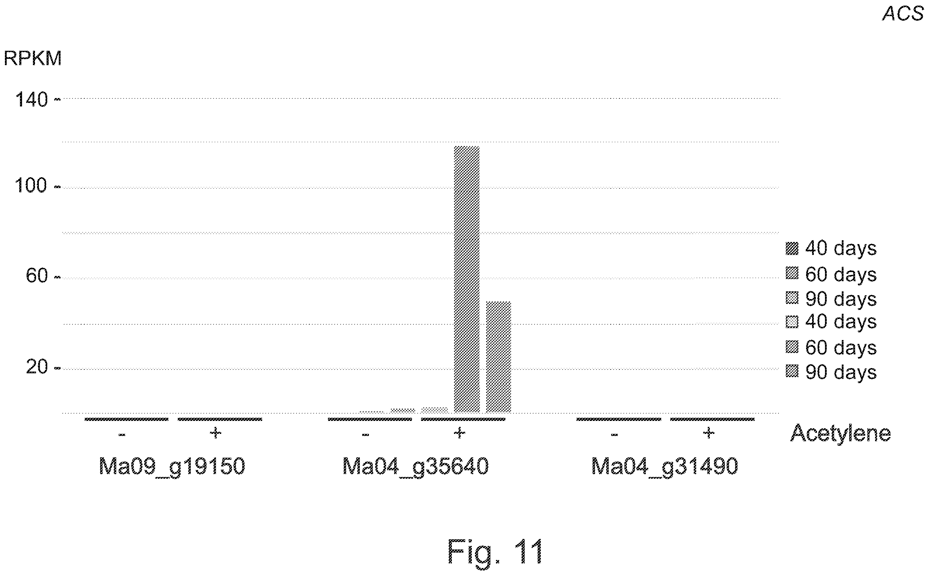

Fig. 11 is a graph showing gene expression of selected *ACS* candidate genes in *M. acuminata* fruits. Experimental conditions are described in D'Hont et al. 2012 Nature. 2012 Aug 9;488(7410):213-7. Fruits were harvested after flowering (40, 60, and 90 days) and kept at 20 °C for 5 days not treated (-) or treated (+) with acetylene to check for transcriptome changes in ripening banana fruits. RNAseq data indicated that acetylene treatment induced changes in gene expression of the banana *ACS* candidate gene *Ma04_g35640.*

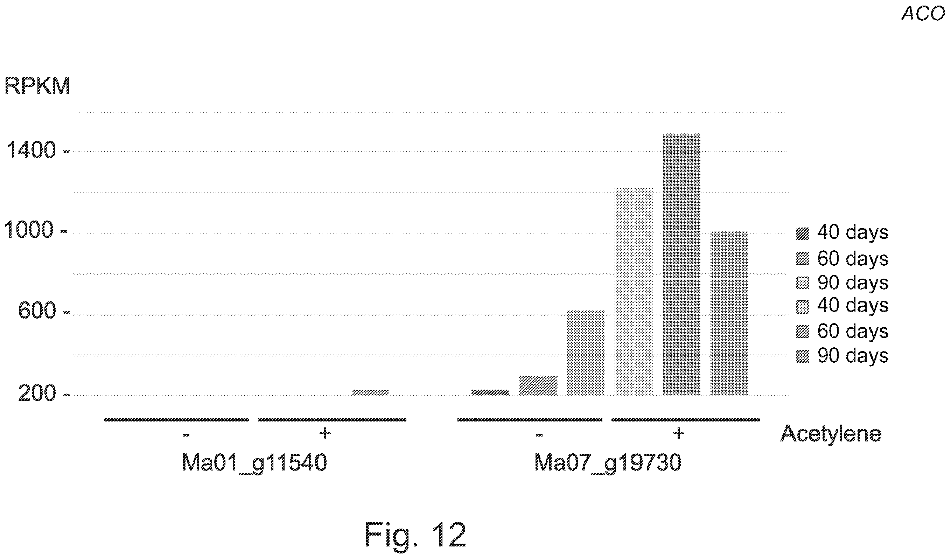

Fig. 12 is a graph showing gene expression of selected *ACO* candidate genes in *M. acuminata* fruits. Experimental conditions are described in D'Hont et al. 2012, supra. Fruits were harvested after flowering (40, 60, and 90 days) and kept at 20 °C for 5 days not treated (-) or treated (+) with acetylene to check for transcriptome changes in ripening banana fruits. RNAseq data indicated that acetylene treatment induced changes in gene expression of the banana *ACO* candidate gene *Ma07_g19730.*

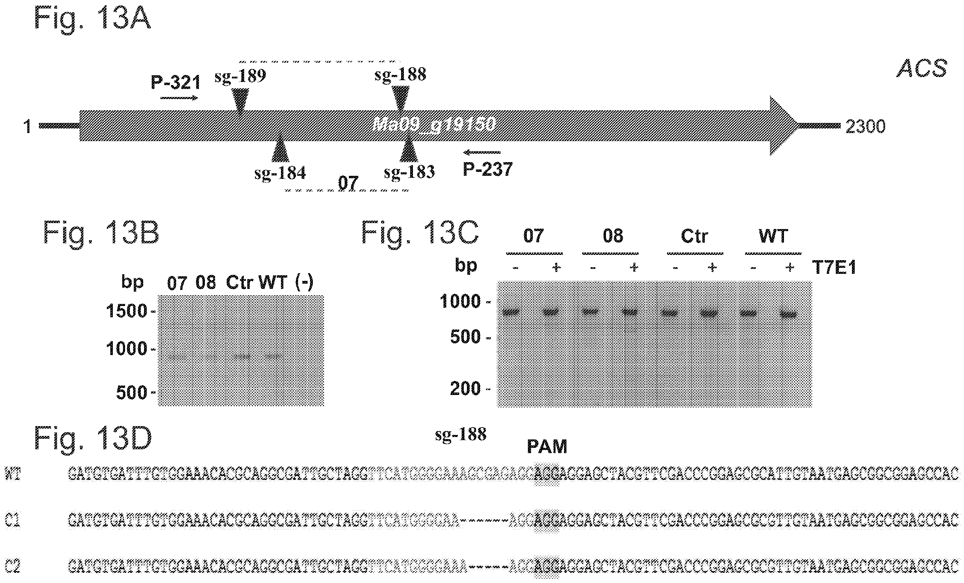

Figs. 13A-D show sequencing analysis and T7 assay revealing the presence of mutations in the candidate gene *Ma09_19150.* (Figure 13A) Cartoon representing the *Ma09_19150* locus indicating the relative positions where the sgRNAs were designed and selected based on conserved regions with other *ACS* genes. (Figure 13B) The *Ma09_19150* locus was amplified with specific primers outside of the sgRNAs region and cloned into pBLUNT (Invitrogen) for sequence analysis and T7E1 assay. (Figure 13C) Mutations detection measured by the T7E1 assay. "Ctr" indicates control plasmid without sgRNAs and WT indicates non-transfected sample (without DNA). 07 and 08 are the combination of the sgRNA used. (Figure 13D) Mutant DNA sequences induced by expression of the genome editing machinery guided by specific sgRNAs are aligned to the wild-type (WT) sequence. The PAM is shown highlighted in grey and the sgRNAs in red letters. Small deletions were found in several clones analyzed.

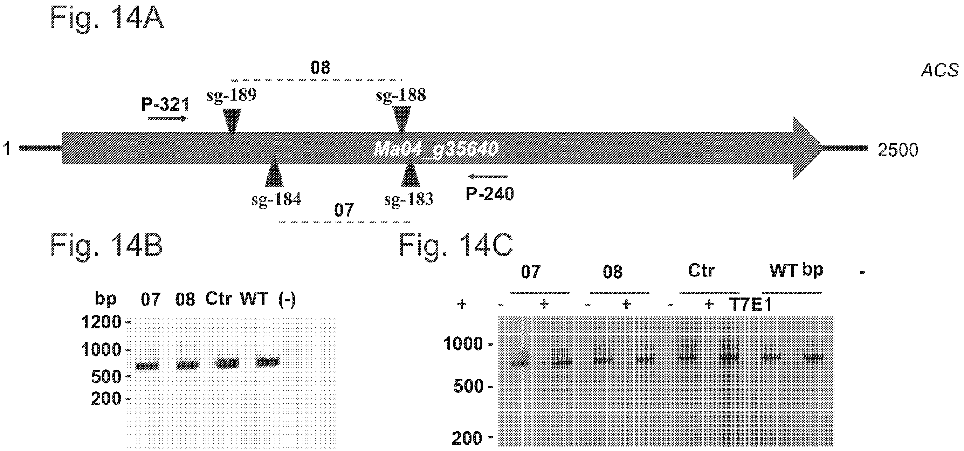

Figs. 14A-C show T7 assay results revealing the presence of mutations in the candidate gene *Ma04_35640.* (Figure 14A) Cartoon representing the *Ma04_35640.* locus indicating the relative positions where the sgRNAs were designed and selected based on conserved regions with other *ACS* genes. (Figure 14B) The *Ma04_35640* locus was amplified with specific primers outside of the sgRNAs region for T7E1 assay. (Figure 14C) Mutations detection measured by the T7E1 assay. "Ctr" indicates control plasmid without sgRNAs and WT indicates non-transfected sample (without DNA). 07 and 08 are the combination of the sgRNA used.

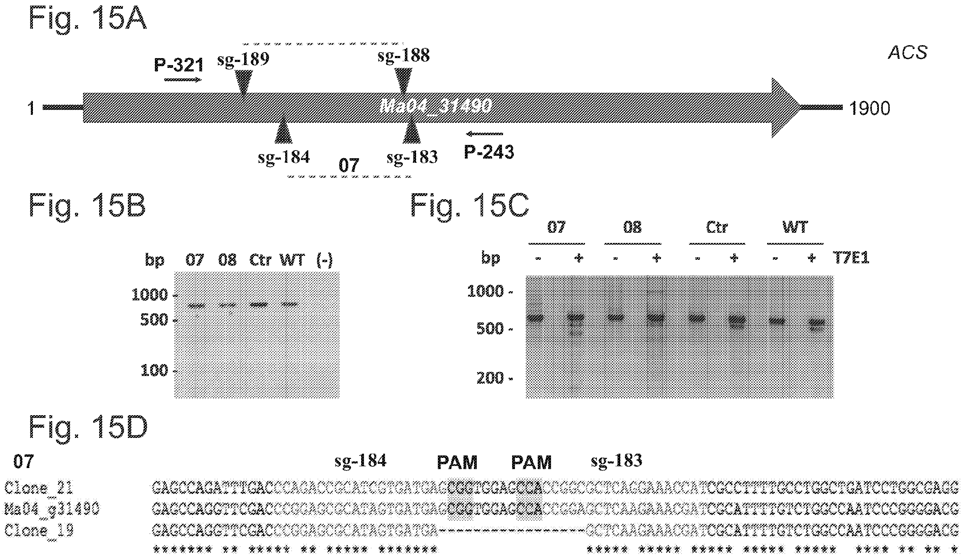

Figs. 15A-D show sequencing analysis and T7 assay revealing the presence of mutations in the candidate gene *Ma04_31490.* (Figure 15A) Cartoon representing the *Ma04_31490* locus indicating the relative positions where the sgRNAs were designed and selected based on conserved regions with other *ACS* genes. (Figure 15B) The *Ma04_31490* locus was amplified with specific primers outside of the sgRNAs region and cloned into pBLUNT (Invitrogen) for sequence analysis and T7E1 assay. (Figure 15C) Mutations detection measured by the T7E1 assay. "Ctr" indicates control plasmid without sgRNAs and WT indicates non-transfected sample (without DNA). 07 and 08 are the combination of the sgRNA used. (Figure 15D) Mutant DNA sequences induced by expression of the genome editing machinery guided by specific sgRNAs are aligned to the wild-type (WT) sequence. The PAM is shown highlighted in grey and the sgRNAs in red letters. WT and small deletions were found in several clones analyzed.

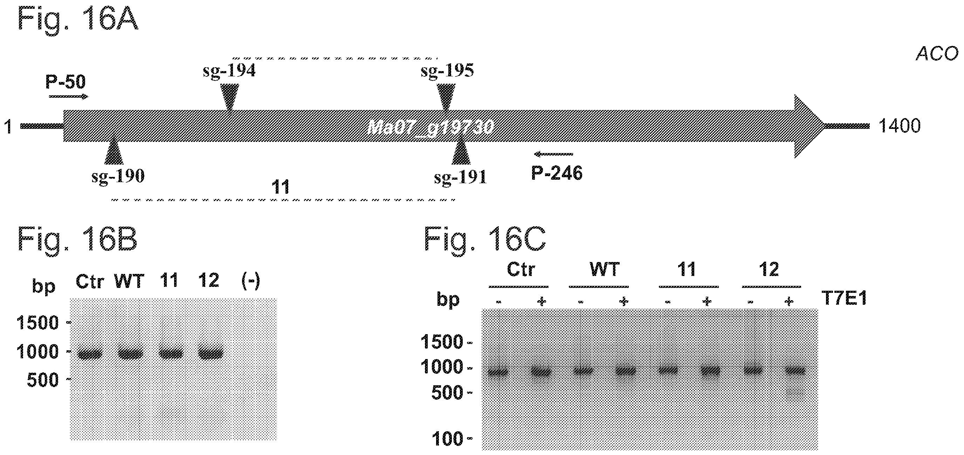

Figs. 16A-C show T7 assay results revealing the presence of mutations in the candidate gene *Ma07_19730.* (Figure 16A) Cartoon representing the *Ma07_19730* locus indicating the relative positions where the sgRNAs were designed and selected based on conserved regions with other *ACO* genes. (Figure 16B) The *Ma07_19730* locus was amplified with specific primers outside of the sgRNAs region for T7E1 assay. (Figure 16C) Mutations detection measured by the T7E1 assay. "Ctr" indicates control plasmid without sgRNAs and WT indicates non-transfected sample (without DNA). 11 and 12 are the combination of the sgRNAs used.

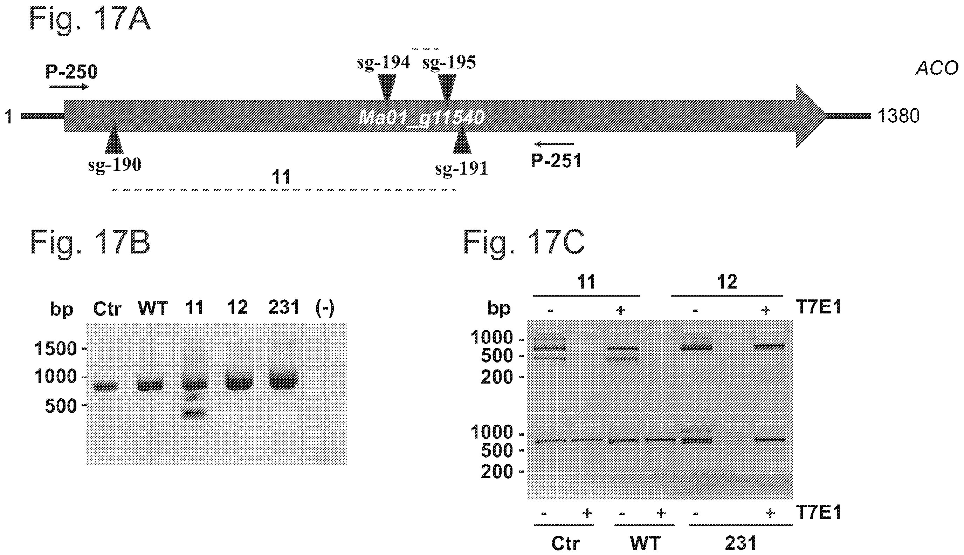

Figs. 17A-C show T7 assay results revealing T7 assay revealed the presence of mutations in the candidate gene *Ma01_11540.* (Figure 17A) Cartoon representing the *Ma01_11540* locus indicating the relative positions where the sgRNAs were designed and selected based on conserved regions with other *ACO* genes. (Figure 17B) The *Ma01_11540* locus was amplified with specific primers outside of the sgRNAs region for T7E1 assay. (Figure 17C) Mutations detection measured by the T7E1 assay. "Ctr" indicates control plasmid without sgRNAs and WT indicates non-transfected sample (without DNA). 11 and 12 are the combination of the sgRNA used and 231 is wildtype gDNA.

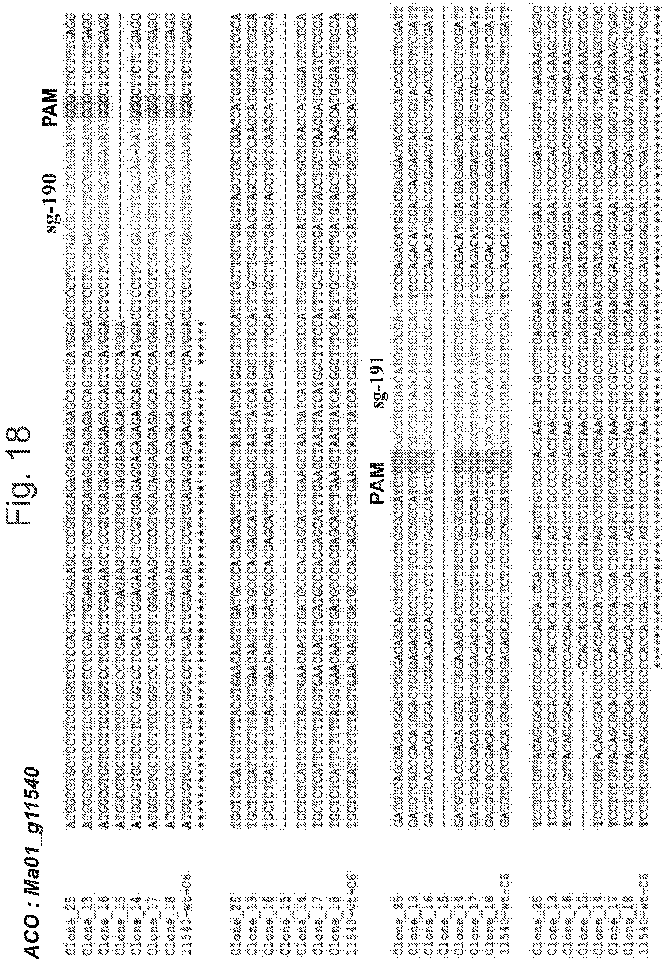

Fig. 18 shows sequencing analysis of mutations in the gene *Ma01_11540.* Mutant DNA sequences induced by expression of the genome editing machinery guided by specific sgRNAs are aligned to the wild-type (WT) sequence. The PAM is shown highlighted in grey and the sgRNAs in red letters. WT and indels were found in several clones analyzed.

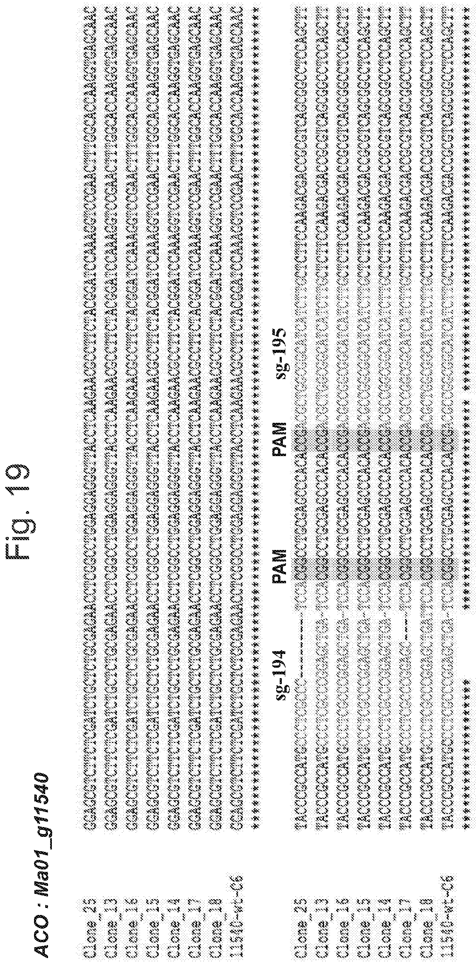

Fig. 19 shows sequencing analysis of mutations in the candidate gene *Ma01_11540.* Mutant DNA sequences induced by expression of the genome editing machinery guided by specific sgRNAs are aligned to the wild-type (WT) sequence. The PAM is shown highlighted in grey, the sgRNAs in red letters, and insertions in green letters. WT and small indels were found in several clones analyzed.

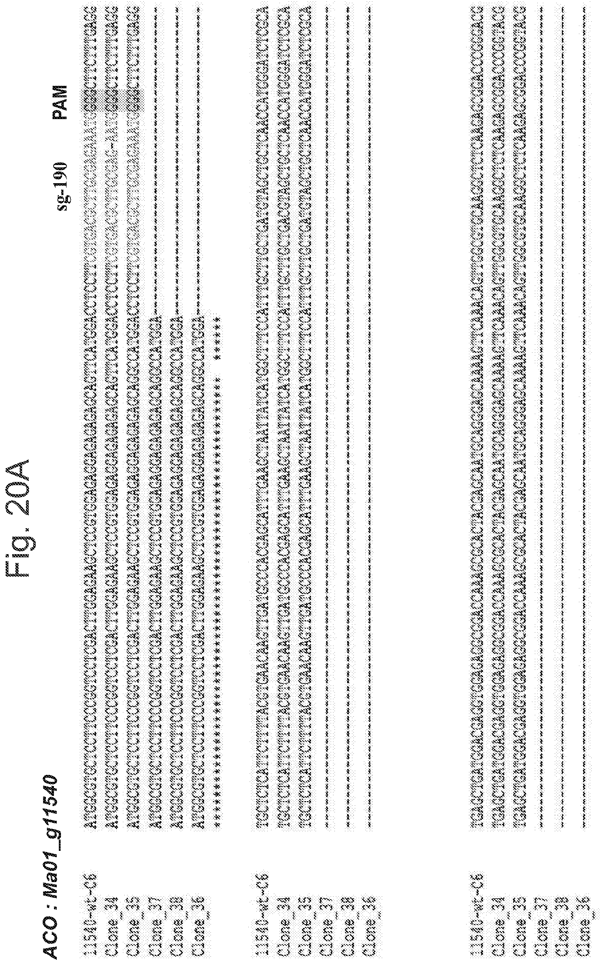

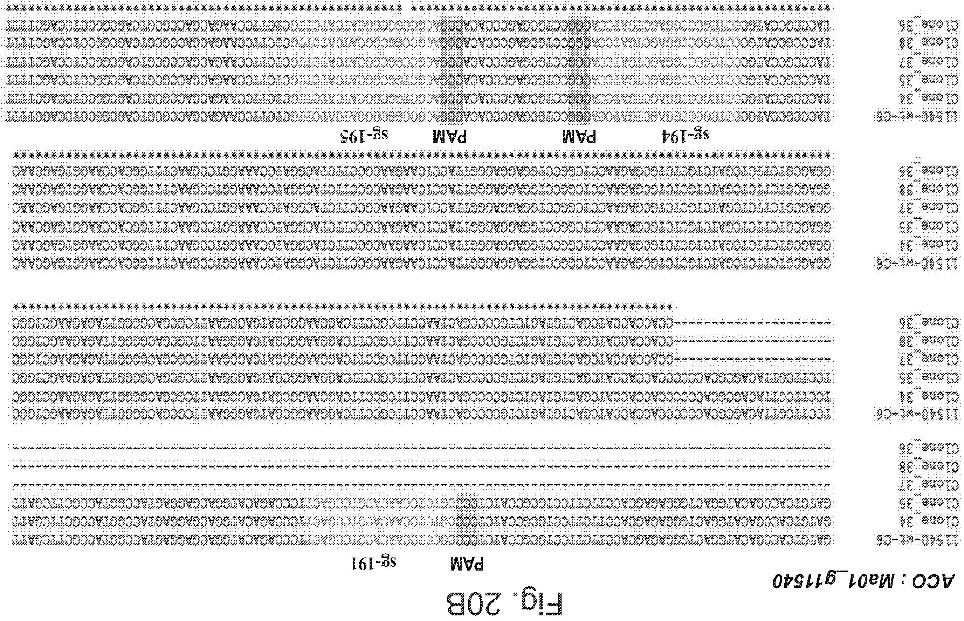

Figs. 20A-B show sequencing analysis of mutations in the candidate gene *Ma01_11540* with various sgRNAs. Mutant DNA sequences induced by expression of the genome editing machinery guided by specific sgRNAs are aligned to the wild-type (WT) sequence. The PAM is shown highlighted in grey and the sgRNAs in red letters. WT sequence, small and large deletions were found in several clones analyzed.

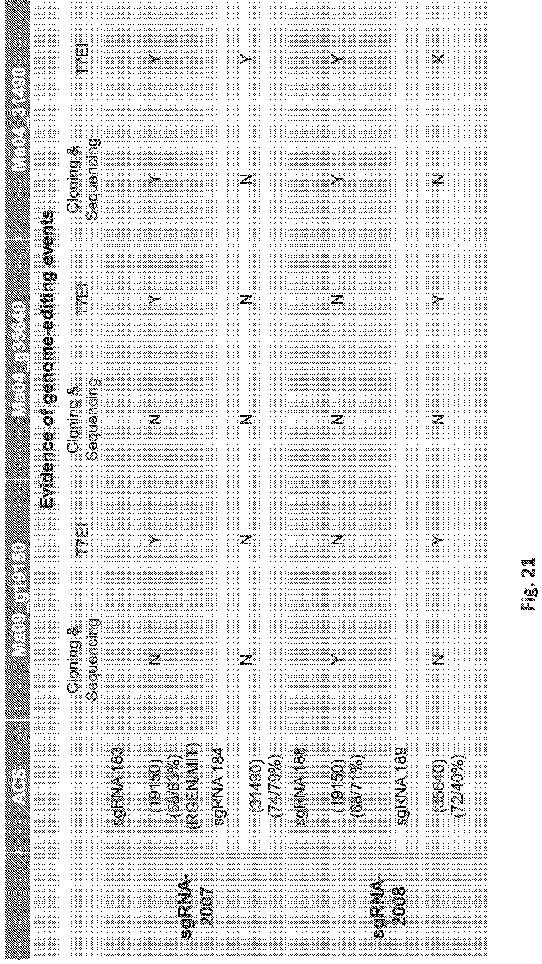

Fig. 21 shows a summary of the evidence of genome-editing events in targeted *ACS* genes. Genome-editing events were assessed by (i) PCR, cloning and sequencing; and (ii) T7EI assay. Y= indels detected; N= no indels detected; X= inconclusive data.

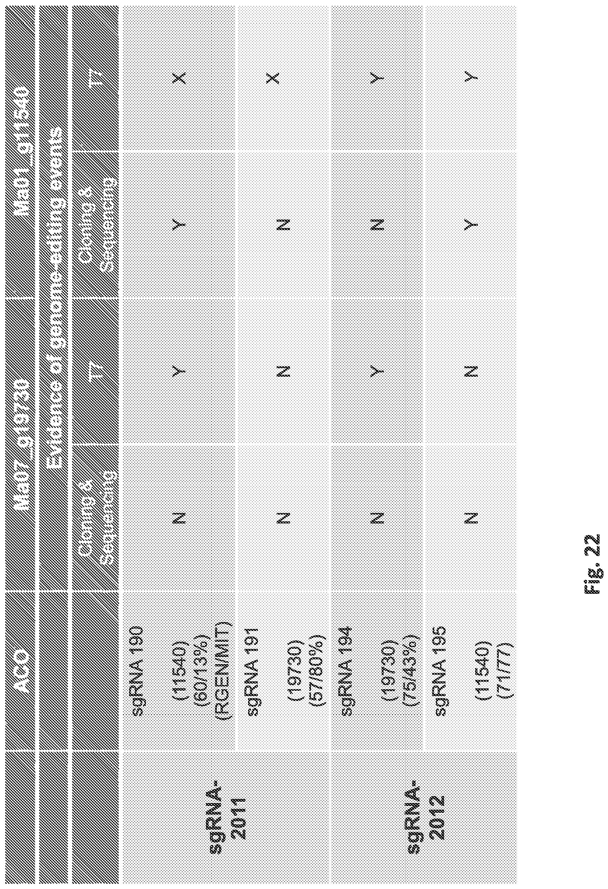

Fig. 22 shows a summary of the evidence of genome-editing events in targeted *ACO* genes. Genome-editing events were assessed by (i) PCR, cloning and sequencing; and (ii) T7EI assay. Y= indels detected; N= no indels detected; X= inconclusive data.

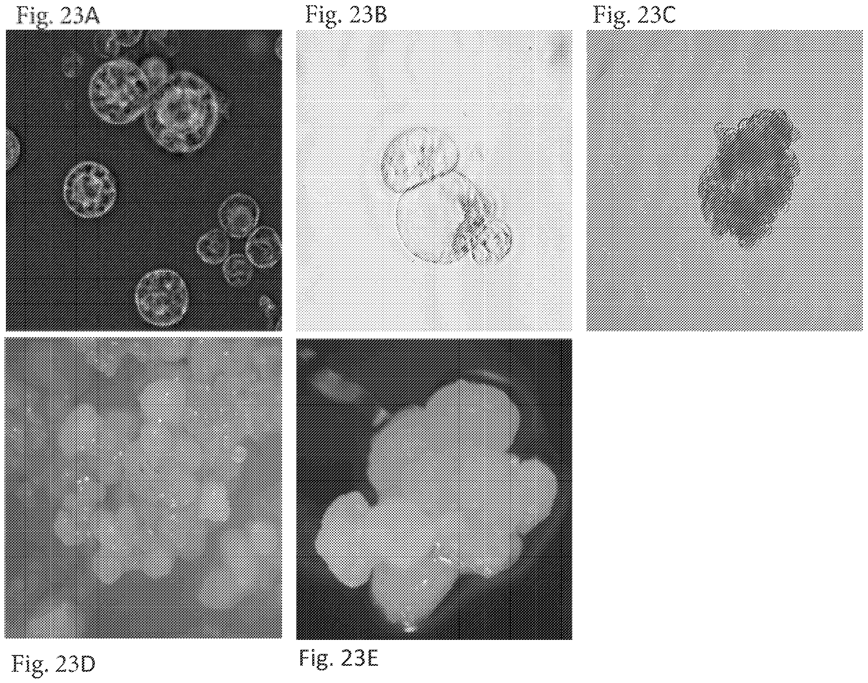

Figs. 23 A-E show transfected banana protoplasts regeneration. Figure 23A. Freshly isolated protoplasts, which were subjected to transfection with plasmids pAC007, pAC2008, pAC2010, pAC2011, or pAC2012. Figure 23B. First cell divisions occur 48h after protoplast isolation and transfection. Figure 23C. Microcalli of embryogenic cells develop after 1-2 months. Figure 23D. Pro-embryos development from embryogenic cells; Figure 23E. Globular embryos; Figure 23F. Regenerated banana plantlets.

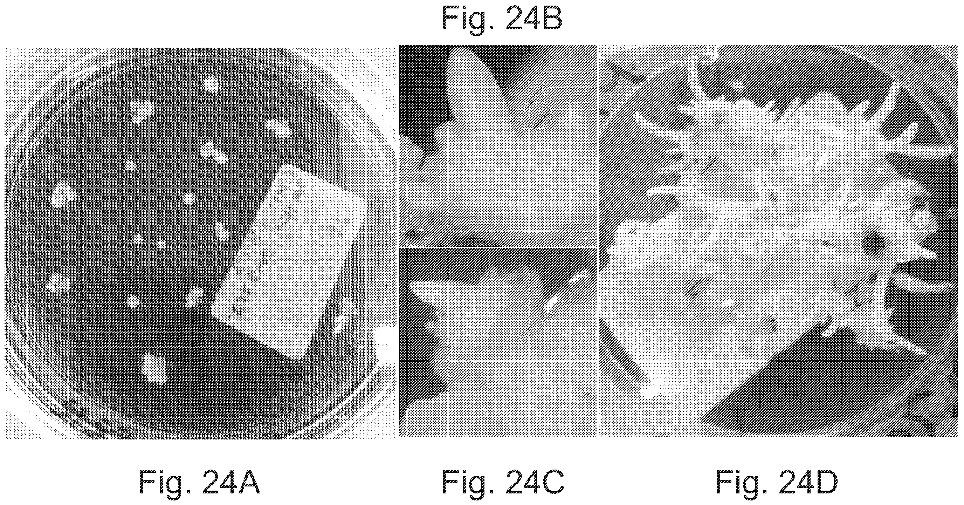

Figs. 24A show regeneration of transfected banana protoplasts. Figure 24A. Mature embryos derived from transfected banana protoplasts in germination medium (GM) containing MS salts and vitamins;
Figs. 24B-C Embryos begin to germinate 1-2 weeks after transfer;
Fig. 24D Germinating embryos 3-4 weeks after transfer to GM (germination medium), ready to be transferred to proliferation medium for shoot elongation.

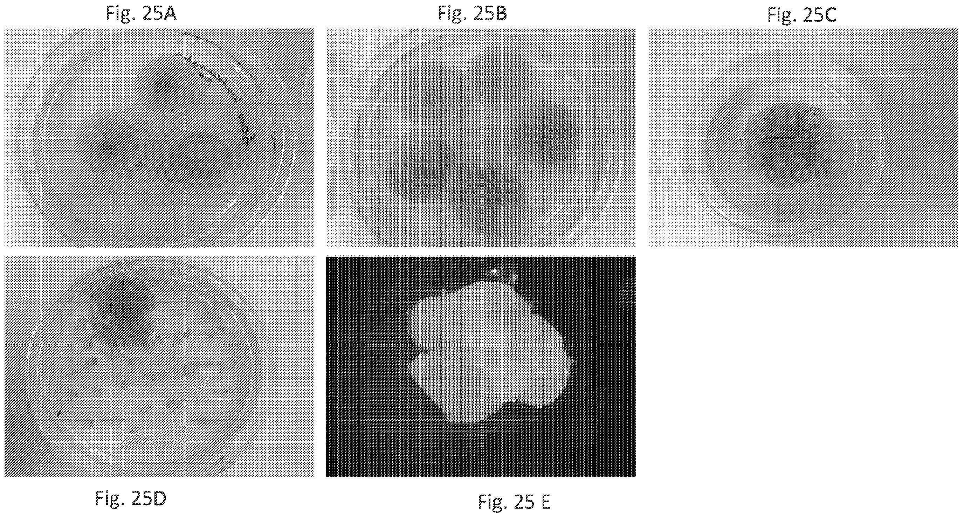

Figs. 25A-E show regeneration of bombarded banana embryogenic cell suspensions (ECS) to extend shelf life. Figure 25A. 3 days old ECS after bombardment on proliferation medium; Figure 25B. Proliferation of bombarded ECS one week after bombardment; Figure 25C. Embryos develop from bombarded ECS, one month after bombardment on embryo development medium (EDM); Figure 25D. Embryos on maturation medium; Figure 25E. Globular embryos.

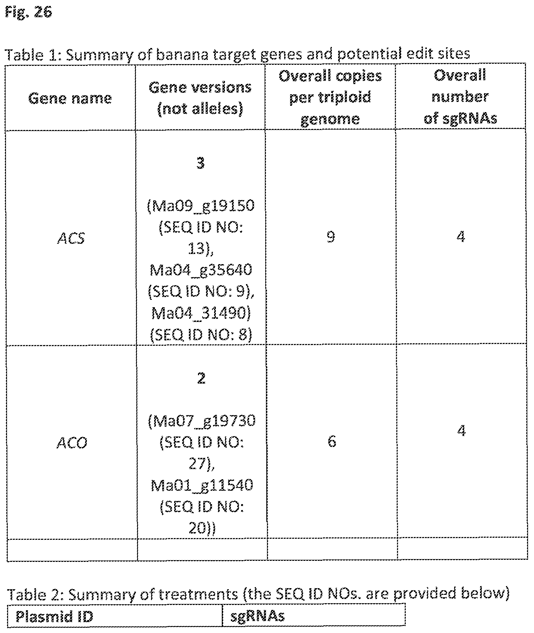

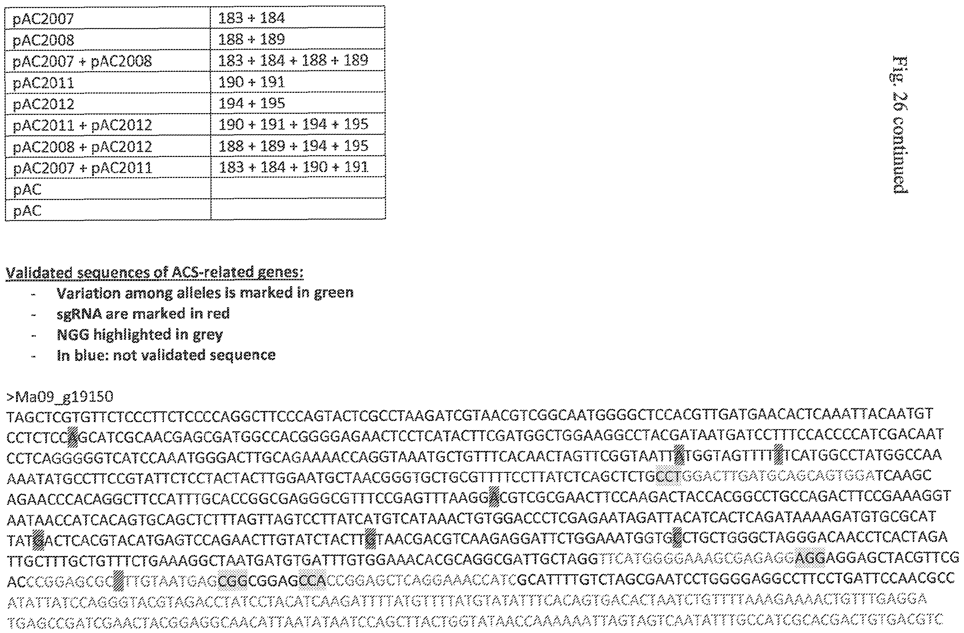

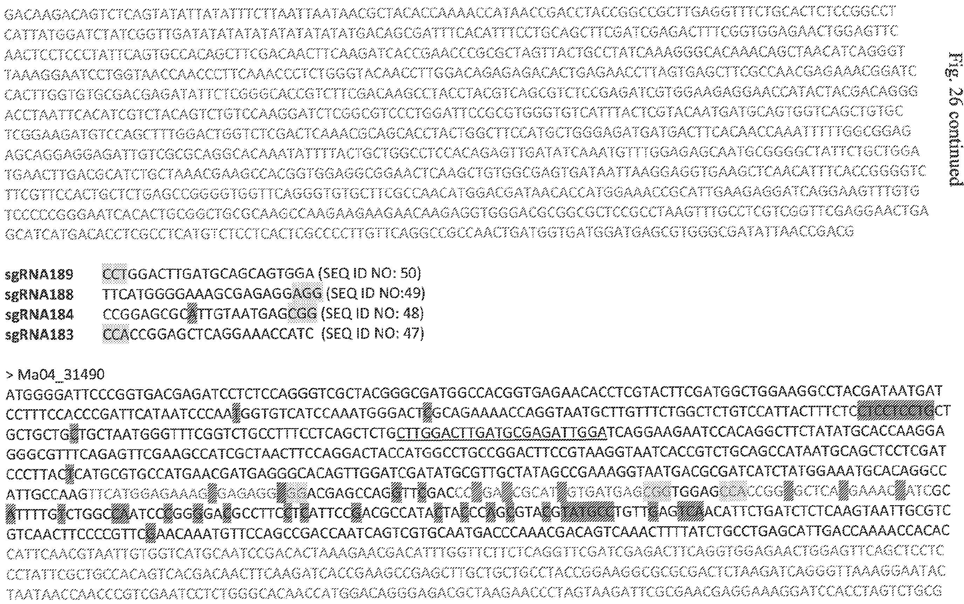

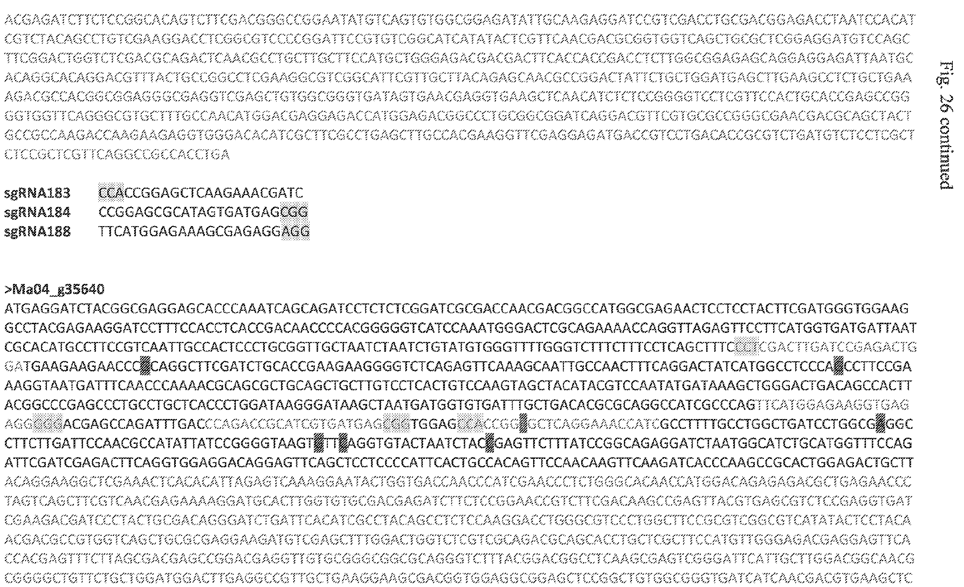

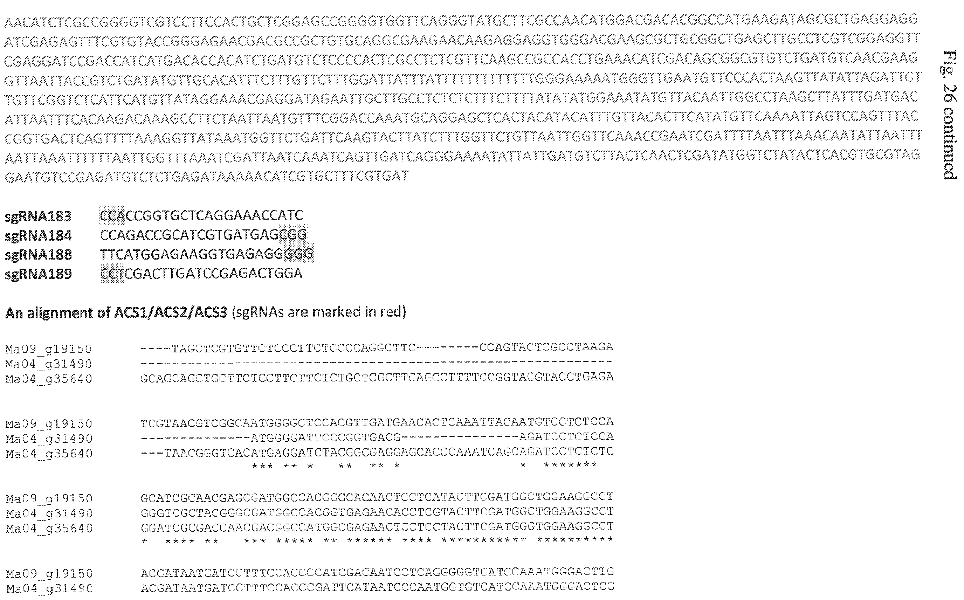

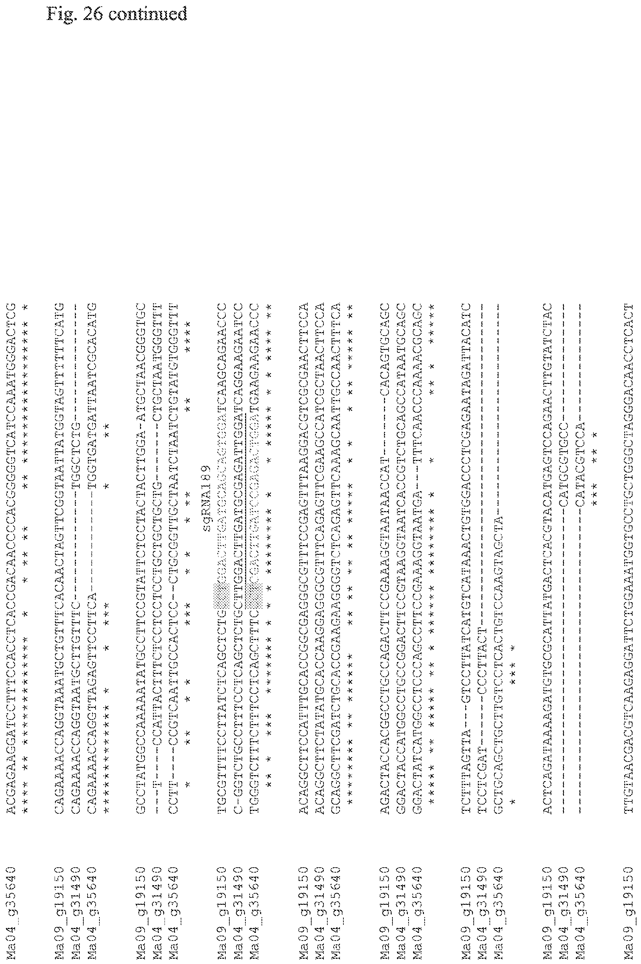

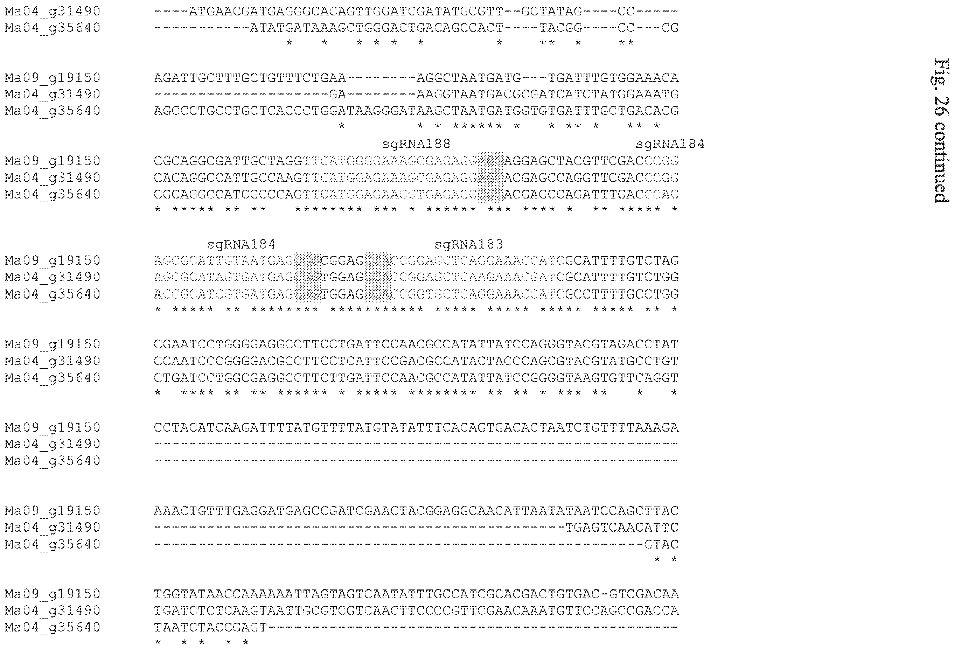

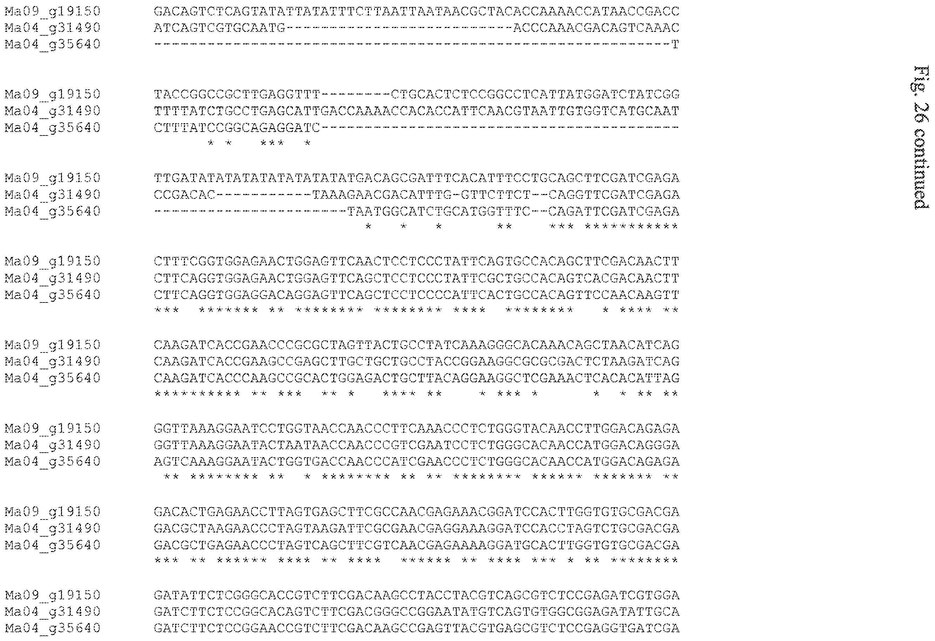

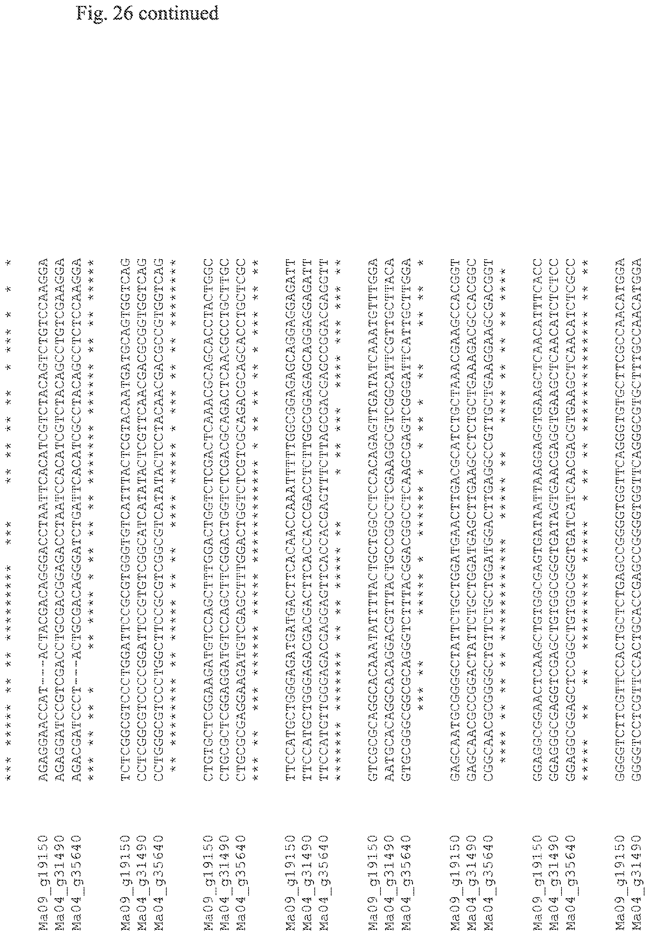

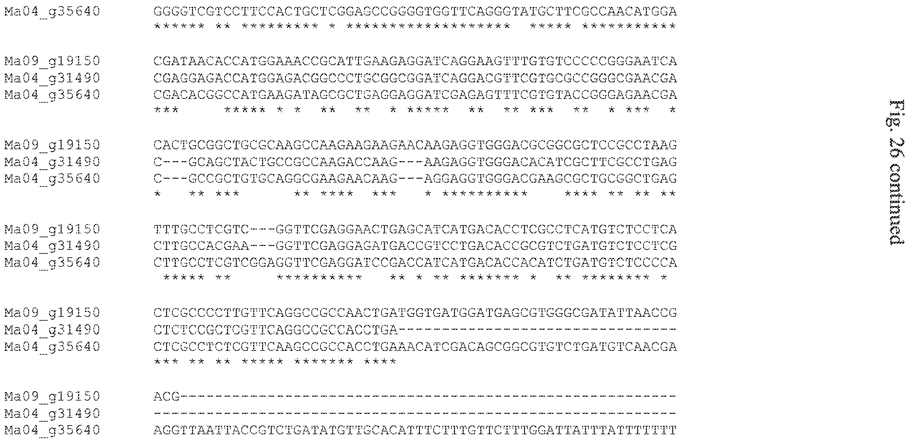

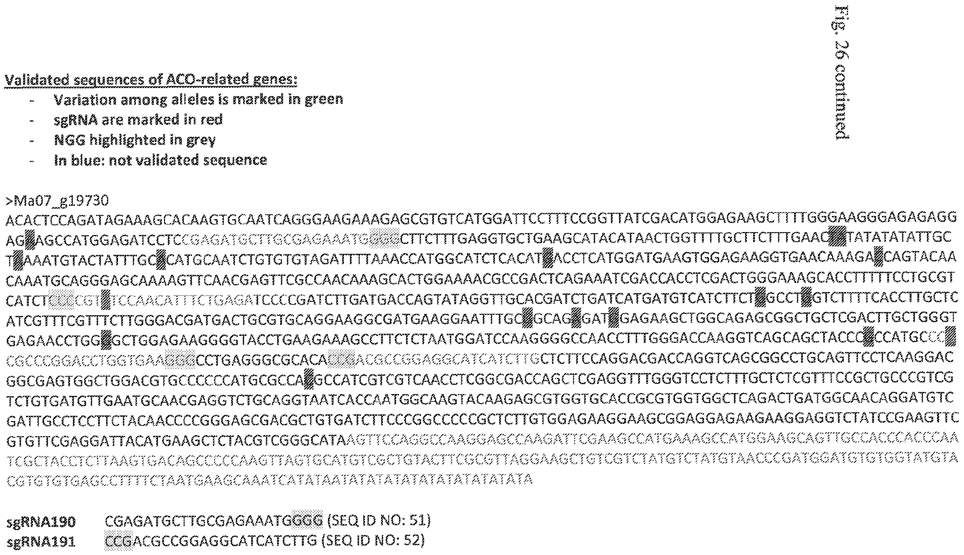

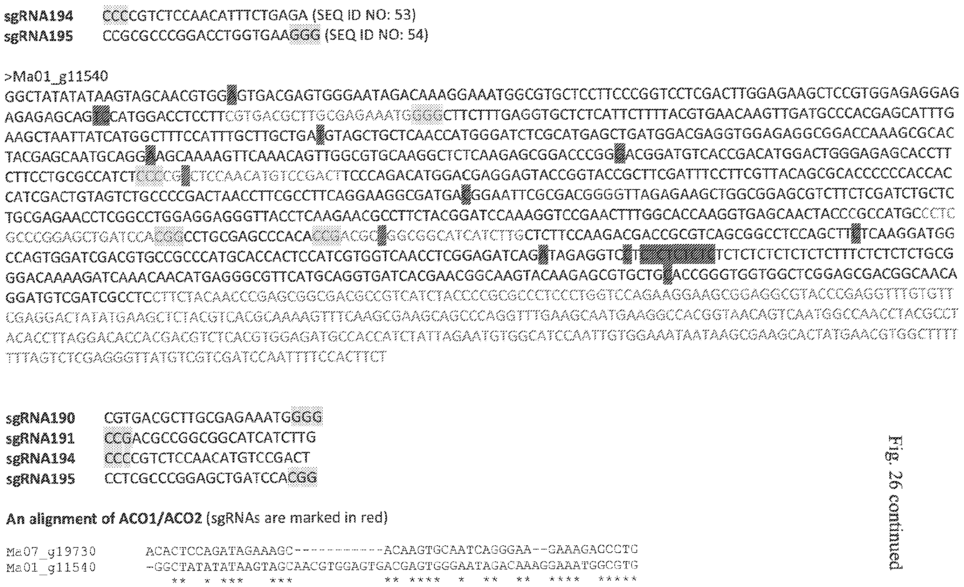

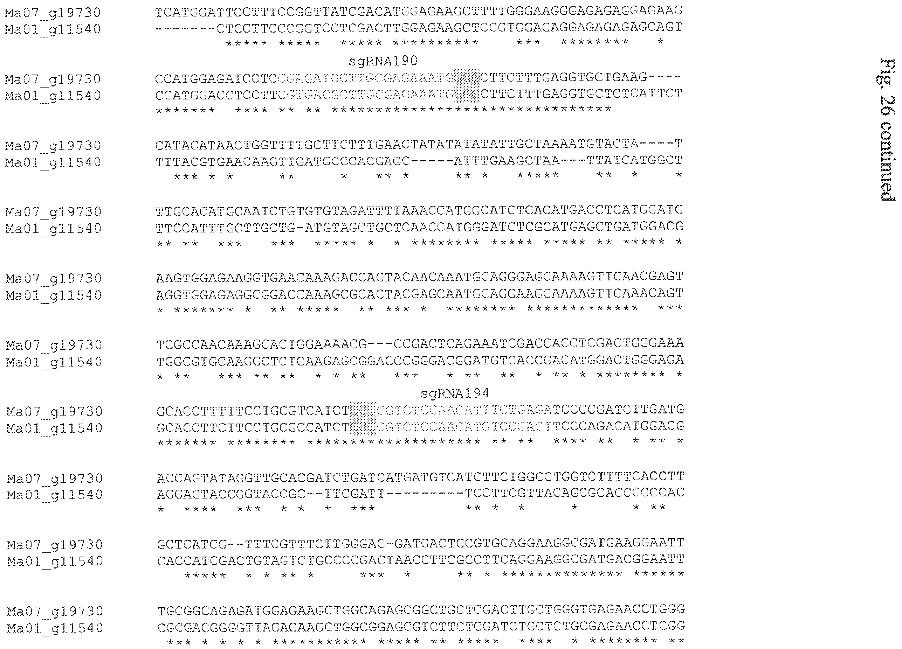

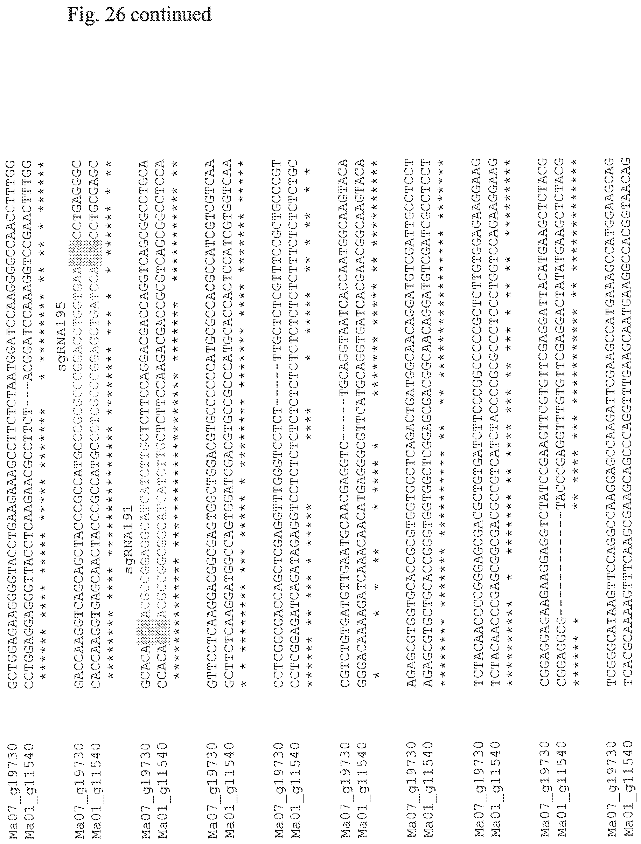

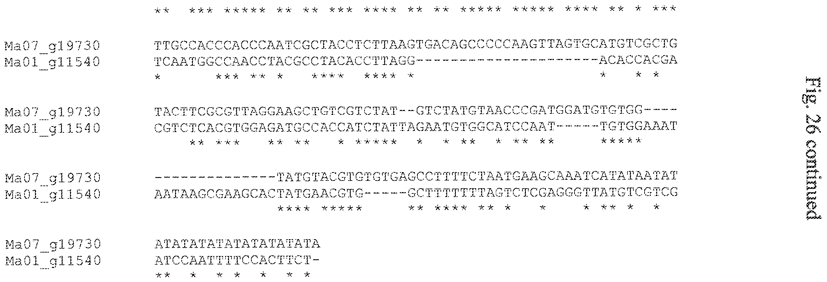

Fig. 26 shows ACO and ACS sequences as well as sgRNAs, sgRNA binding sites and primers used according to some embodiments of the invention. Red highlight denotes the positions of the sgRNAs along the targeted sequences; Color code is provided in the figure.

### DESCRIPTION OF SPECIFIC EMBODIMENTS OF THE INVENTION

The present invention, in some embodiments thereof, relates to compositions and methods for increasing shelf-life of banana.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Ethylene, the simplest unsaturated hydrocarbon (two carbons with a double bond) is a gaseous plant hormone which regulates essentially all physiological processes during the plant's life cycle. It is responsible for signaling changes in: seed dormancy and germination, root growth and nodulation, shoot and leaf formation, flower and fruit development, different organs senescence and abscission, plant defense mechanisms, and a number of interactions with other plant hormones. Although, ethylene is undoubtedly essential for proper plant growth, development, and survival, it may also be deleterious to plants in some instances. Increased ethylene levels in plants exposed to various types of stress including chilling, heat, nutrient deprivation, anaerobiosis, wounding, and pathogen infection with increased damage to plant growth and health as the result has been reported. There is thus a considerable commercial interest in genetically modifying the amount of ethylene produced under ripening, senescing or stress conditions and thereby creating plants with more robust and/or desirable trait

The most established method of plant genetic engineering using CRISPR-Cas genome editing technology requires the insertion of new DNA into the host's genome. This insert, a transfer DNA (T-DNA), carries several transcriptional units in order to achieve successful CRISPR-Cas-mediated genome edits. These commonly consist of an antibiotic resistance gene to select for transgenic plants, the Cas machinery, and several sgRNA units. Because of the integration of foreign DNA into the genome, plants generated this way are classified as transgenic or genetically modified (GM). Once a genome edit has been established in the host, the T-DNA can be removed through sexual propagation and breeding, as the CRISPR Cas9 machinery is no longer needed to maintain the phenotype. However, for parthenocarpic crops, such as banana, that do not produce viable seeds, removal of T-DNA by sexual reproduction is impossible.

Embodiments of the invention relate to the identification of targets for genome editing in the ethylene biosynthesis pathway of the banana.

Thus, to reduce ethylene levels in banana plants, which may result in extended shelf-life of banana fruits, knockout of genes involved in the biosynthesis of ethylene, including ACS and ACO (Figure 7A, 7B) was attempted. However, the banana genome contains multiple sequences that are homologous to these genes.

In order to identify superior target genes within the banana genome, which encode functional ACS and ACO, homologous sequences from characterized pathways in model or crop species were identified. The process involved a series of sequential steps for comparative analysis of DNA and protein sequences that aim at reconstructing the evolutionary history of genes through phylogenetic analysis, filtering candidates by validating their expression in general and target tissue, and sequencing of candidate genes to ensure appropriate sgRNA design (to avoid mismatches). This procedure allowed the selection of genes, the identification of optimized target regions for knockout (conserved and potentially catalytic domains) and the design of appropriate sgRNAs.

Following transfection of banana protoplasts with sgRNAs directed at a plurality of genes in the ethylene biosynthesis pathway, the present inventors were able to identify robust genome editing in key genes e.g., Ma07_g19730 and Ma04_g35640 as well as in other genes of the families to avoid compensation by redundancy. Such protoplasts were also subjected to regeneration protocols so as to obtain a banana plant having a long shelf-life (see Figures 8-25A-E).

Thus, according to as aspect there is provided a method of increasing shelf-life of banana, the method comprising:
(a) subjecting a banana plant cell to a DNA editing agent directed at a nucleic acid sequence encoding a component in an ethylene biosynthesis pathway of the banana to result in a loss of function mutation in said nucleic acid sequence encoding said ethylene biosynthesis pathway and
(b) regenerating a plant from said plant cell.

As used herein the term "banana" refers to a plant of the genus Musa, including Plantains.

According to a specific embodiment, the banana is triploid.

Other ploidies are also contemplated, including, diploid and tetraploid.

As used herein "plant" refers to whole plant(s), a grafted plant, ancestors and progeny of the plants and plant parts, including seeds, fruits, shoots, stems, roots (including tubers), rootstock, scion, and plant cells, tissues and organs.

According to a specific embodiment, the plant part is a fruit.

According to a specific embodiment, the plant part is a seed.

""Seed," refers to a flowering plant's unit of reproduction, capable of developing into another such plant.

According to a specific embodiment, the cell is a germ cell.

According to a specific embodiment, the cell is a somatic cell.

The plant may be in any form including suspension cultures, protoplasts, embryos, meristematic regions, callus tissue, leaves, gametophytes, sporophytes, pollen, and microspores.

According to a specific embodiment, the plant part comprises DNA.

Following is a non-limiting list of cultivars that can be used according to the present teachings.
AA Group
   Diploid *Musa acuminata,* both wild banana plants and cultivars
   Chingan banana
   Lacatan banana
   Lady Finger banana (Sugar banana)
   Pisang jari buaya (Crocodile fingers banana)
   Señorita banana (Monkoy, Arnibal banana, Cuarenta dias, Cariñosa, Pisang Empat Puluh Hari, Pisang Lampung)^{[12]}
   Sinwobogi banana
AAA Group
   Triploid *Musa acuminata,* both wild banana plants and cultivars
   Cavendish Subgroup
   'Dwarf Cavendish'
   'Giant Cavendish' ('Williams')
   'Grand Nain' ('Chiquita')
   'Masak Hijau'
   'Robusta'
   'Red Dacca'
   Dwarf Red banana
   Gros Michel banana
   East African Highland bananas (AAA-EA subgroup)
AAAA Group
   Tetraploid *Musa acuminata,* both wild bananas and cultivars
   Bodies Altafort banana
   Golden Beauty banana
AAAB Group
   Tetraploid cultivars of *Musa* × *paradisiaca*
   Atan banana
   Goldfinger banana
AAB Group

Triploid cultivars of *Musa* × *paradisiaca.* This group contains the Plantain subgroup, composed of "true" plantains or African Plantains - whose centre of diversity is Central and West Africa, where a large number of cultivars were domesticated following the introduction of ancestral Plantains from Asia, possibly 2000-3000 years ago.
The Iholena and Maoli-Popo'ulu subgroups are referred to as Pacific plantains.
Iholena subgroup - *subgroup of cooking bananas domesticated in the Pacific region*
Maoli-Popo'ulu subgroup - *subgroup of cooking bananas domesticated in the Pacific region* Maqueño banana
Popoulu banana
Mysore subgroup - *cooking and dessert bananas*^{[15]}
Mysore banana
Pisang Raja subgroup
Pisang Raja banana
Plantain subgroup
French plantain
Green French banana
Horn plantain & Rhino Horn banana
Nendran banana
Pink French banana
Tiger banana
Pome subgroup
Pome banana
Prata-anã banana (Dwarf Brazilian banana, Dwarf Prata)
Silk subgroup
Latundan banana (Silk banana, Apple banana)
Others
Pisang Seribu banana
plu banana
   AABB Group
Tetraploid cultivars of *Musa* × *paradisiaca*
Kalamagol banana
Pisang Awak (Ducasse banana)
   AB Group
Diploid cultivars of *Musa* × *paradisiaca*
Ney Poovan banana
   ABB Group
Triploid cultivars of *Musa* × *paradisiaca*
Blue Java banana (Ice Cream banana, Ney mannan, Ash plantain, Pata hina, Dukuru, Vata)
Bluggoe Subgroup
Bluggoe banana (also known as orinoco and "burro")
Silver Bluggoe banana
Pelipita banana (Pelipia, Pilipia)
Saba Subgroup
Saba banana (Cardaba, Dippig)
Cardaba banana
Benedetta banana
   ABBB Group
Tetraploid cultivars of *Musa* × *paradisiaca*
Tiparot banana
   BB Group
Diploid *Musa balbisiana,* wild bananas
   BBB Group
Triploid *Musa balbisiana,* wild bananas and cultivars
Kluai Lep Chang Kut

According to a specific embodiment, the plant is a plant cell e.g., plant cell in an embryonic cell suspension.

According to a specific embodiment, the plant cell is a protoplast.

The protoplasts are derived from any plant tissue e.g., roots, leaves, embryonic cell suspension, calli or seedling tissue.

As used herein "component in the ethylene biosynthesis pathway" refers to a polypeptide that is essential for ethylene biosynthesis in banana e.g., an enzyme. Specifically, ethylene biosynthesis begins from S-adenosylmethionine (SAM) and includes two key steps (Figure 1) as reviewed by Pech et al. (2010, Ethylene biosynthesis. In: Plant hormones: biosynthesis, transduction, action, 3rd edn. Springer, Dordrecht, pp 115-136).

The biosynthesis pathway of ethylene comprises of conversion of methionine into S-Adenosyl-methionine (AdoMet, SAM) with the aid of AdoMet synthase. 1-aminocyclopropane-1-carboxylate synthase (ACS) [EC 4.4.1.14] catalyses the cyclization of SAM to 1-aminocyclopropane-1-carboxylic acid (ACC), which is often considered the rate-limiting reaction in the pathway. ACS also produces 5'-methylthioadenosine (MTA) which is recycled to regenerate methionine. The final step, oxygen-dependent conversion of ACC to ethylene, is catalyzed by ACC oxidase (ACO) [EC 1.14.17.4]. ACC is converted to ethylene by a modification of carbons C-2 and C-3 of ACC, while C-1 is converted to cyanide and the carboxyl group converted into carbon dioxide.

According to a specific embodiment, the AdoMet synthase is banana AdoMet.

All accession numbers correspond to the publicly available genome M. acuminata doubled-haploid of the germplasm collection accession named Pahang (2n=22) assembly version 2.

All accession numbers correspond to the publicly available genome M. acuminata doubled-haploid of the germplasm collection accession named Pahang (2n=22) assembly version 2.

According to a specific embodiment, the ACS is:
>Ma01_g07800.1 (SEQ ID NO: 1
>Ma01_g12130.1 (SEQ ID NO: 2);
>Ma02_g10500.1 (SEQ ID NO: 3);
>Ma03_g12030.1 (SEQ ID NO: 4);
>Ma03_g27050.1 (SEQ ID NO: 5);
>Ma04_g01260.1 (SEQ ID NO: 6);
>Ma04_g24230.1 (SEQ ID NO: 7);
>Ma04_g31490.1 (SEQ ID NO: 8);
>Ma04_g35640.1 (SEQ ID NO: 9);
>Ma04_g37400.1 (SEQ ID NO: 10);
>Ma05_g08580.1 (SEQ ID NO: 11);
>Ma05_g13700.1 (SEQ ID NO: 12);
>Ma09_g19150.1 (SEQ ID NO: 13); or
>Ma10_g27510.1 (SEQ ID NO: 14);

According to a specific embodiment, the ACO is
>Ma09_g04370.1 (SEQ ID NO: 15);
>Ma06_g17160.1 (SEQ ID NO: 16);
>Ma11_g05490.1 (SEQ ID NO: 17);
>Ma00_g04490.1 (SEQ ID NO: 18);
>Ma07_g15430.1 (SEQ ID NO: 19);
>Ma01_g 11540.1 (SEQ ID NO: 20);
>Ma10_g16100.1 (SEQ ID NO: 21);
>Ma05_g08170.1 (SEQ ID NO: 22);
>Ma06_g14430.1 (SEQ ID NO: 23);
>Ma05_g09360.1 (SEQ ID NO: 24);
>Ma11_g22170.1 (SEQ ID NO: 25);
>Ma05_g31690.1 (SEQ ID NO: 26);
>Ma07_g19730.1 (SEQ ID NO: 27);
>Ma06_g02600.1 (SEQ ID NO: 28);
>Ma10_g05270.1 (SEQ ID NO: 29);
>Ma06_g14370.1 (SEQ ID NO: 30);
>Ma11_g05480.1 (SEQ ID NO: 31);
>Ma06_g14410.1 (SEQ ID NO: 32);
>Ma06_g14420.1 (SEQ ID NO: 33);
>Ma06_g34590.1 (SEQ ID NO: 34);
>Ma02_g21040.1 (SEQ ID NO: 35);
>Ma11_g04210.1 (SEQ ID NO: 36);
>Ma05_g12600.1 (SEQ ID NO: 37);
>Ma04_g23390.2 (SEQ ID NO: 38);
>Ma03_g06970.1 (SEQ ID NO: 39);
>Ma05_g09980.1 (SEQ ID NO: 40);
>Ma04_g36640.1 (SEQ ID NO: 41);
>Ma11_g04180.1 (SEQ ID NO: 42);
>Ma11_g02650.1 (SEQ ID NO: 43); or
>Ma00_g04770.1 (SEQ ID NO: 44);

According to a specific embodiment, the ACO is Ma01_g11540.1 (SEQ ID NO: 20) and/or Ma07_g19730.1 (SEQ ID NO: 27):
According to a specific embodiment, the ACS is Ma09_g19150.1 (SEQ ID NO: 13), Ma04_g35640.1 (SEQ ID NO: 9) and/or Ma04_g31490.1 (SEQ ID NO: 8):
Also contemplated are naturally occurring functional homologs of each of the above genes e.g., exhibiting at least 80 %, 81 %, 82 %, 83 %, 84 %, 85 %, 86 %, 87 %, 88 %, 89 %, 90 %, 91 %, 92 %, 93 %, 94 %, 95 %, 96 %, 97 % 98 % or 99 % identity to the above-mentioned genes and having an ACS or ACO activity, as defined above.

As used herein, "sequence identity" or "identity" or grammatical equivalents as used herein in the context of two nucleic acid or polypeptide sequences includes reference to the residues in the two sequences which are the same when aligned. When percentage of sequence identity is used in reference to proteins it is recognized that residue positions which are not identical often differ by conservative amino acid substitutions, where amino acid residues are substituted for other amino acid residues with similar chemical properties (*e.g.* charge or hydrophobicity) and therefore do not change the functional properties of the molecule. Where sequences differ in conservative substitutions, the percent sequence identity may be adjusted upwards to correct for the conservative nature of the substitution. Sequences which differ by such conservative substitutions are considered to have "sequence similarity" or "similarity". Means for making this adjustment are well-known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, *e.g.,* according to the algorithm of Henikoff S and Henikoff JG. [Amino acid substitution matrices from protein blocks. Proc. Natl. Acad. Sci. U.S.A. 1992, 89(22): 10915-9].

Identity can be determined using any homology comparison software, including for example, the BlastN software of the National Center of Biotechnology Information (NCBI) such as by using default parameters.

According to some embodiments of the invention, the identity is a global identity, *i.e.,* an identity over the entire nucleic acid sequences of the invention and not over portions thereof.

As used herein "plant" refers to whole plant(s), a grafted plant, ancestors and progeny of the plants and plant parts, including seeds, fruits, shoots, stems, roots (including tubers), rootstock, scion, and plant cells, tissues and organs.

The plant may be in any form including suspension cultures, protoplasts, embryos, meristematic regions, callus tissue, leaves, gametophytes, sporophytes, pollen, and microspores.

According to a specific embodiment, the plant part comprises DNA.

According to a specific embodiment, the banana plant is of a banana breeding line, more preferably an elite line.

According to a specific embodiment, the banana plant is of an elite line.

According to a specific embodiment, the banana plant is of a purebred line.

According to a specific embodiment, the banana plant is of a banana variety or breeding germplasm.

The term "breeding line", as used herein, refers to a line of a cultivated banana having commercially valuable or agronomically desirable characteristics, as opposed to wild varieties or landraces. The term includes reference to an elite breeding line or elite line, which represents an essentially homozygous, usually inbred, line of plants used to produce commercial F₁ hybrids. An elite breeding line is obtained by breeding and selection for superior agronomic performance comprising a multitude of agronomically desirable traits. An elite plant is any plant from an elite line. Superior agronomic performance refers to a desired combination of agronomically desirable traits as defined herein, wherein it is desirable that the majority, preferably all of the agronomically desirable traits are improved in the elite breeding line as compared to a non-elite breeding line. Elite breeding lines are essentially homozygous and are preferably inbred lines.

The term "elite line", as used herein, refers to any line that has resulted from breeding and selection for superior agronomic performance. An elite line preferably is a line that has multiple, preferably at least 3, 4, 5, 6 or more (genes for) desirable agronomic traits as defined herein.

The terms "cultivar" and "variety" are used interchangeable herein and denote a plant with has deliberately been developed by breeding, e.g., crossing and selection, for the purpose of being commercialized, e.g., used by farmers and growers, to produce agricultural products for own consumption or for commercialization. The term "breeding germplasm" denotes a plant having a biological status other than a "wild" status, which "wild" status indicates the original non-cultivated, or natural state of a plant or accession.

The term "breeding germplasm" includes, but is not limited to, semi-natural, semi-wild, weedy, traditional cultivar, landrace, breeding material, research material, breeder's line, synthetic population, hybrid, founder stock/base population, inbred line (parent of hybrid cultivar), segregating population, mutant/genetic stock, market class and advanced/improved cultivar. As used herein, the terms "purebred", "pure inbred" or "inbred" are interchangeable and refer to a substantially homozygous plant or plant line obtained by repeated selfing and-or backcrossing.

As used herein "modifying a genome" refers to introducing at least one mutation in at least one allele encoding a component in the ethylene biosynthesis pathway in banana. According to some embodiments, modifying refers to introducing a mutation in each allele of a component in the ethylene biosynthesis pathway. According to at least some embodiments, the mutation on the two alleles of the component in the ethylene biosynthesis pathway is in a homozygous form.

According to some embodiments, mutations on the two alleles encoding the component in the ethylene biosynthesis pathway are noncomplementary.

According to a specific embodiment, the DNA editing agent modifies the target sequence of the component in the ethylene biosynthesis pathway and is devoid of "off target" activity, i.e., does not modify other sequences in the banana genome.

According to a specific embodiment, the DNA editing agent comprises an "off target activity" on a non-essential gene in the banana genome.

Non-essential refers to a gene that when modified with the DNA editing agent does not affect the phenotype of the target genome in an agriculturally valuable manner (e.g., nutritional value, flavor, biomass, yield, biotic/abiotic stress tolerance and the like).

Off-target effects can be assayed using methods which are well known in the art and are described herein.

As used herein "loss of function" mutation refers to a genomic aberration which results in reduced ability (i.e., impaired function) or inability of the component of the ethylene biosynthesis pathway to facilitate in the synthesis of ethylene or precursor thereof.

As used herein "reduced ability" refers to reduced activity of the component in the ethylene biosynthesis pathway activity (i.e., synthesis of ethylene) as compared to that of the wild-type enzyme devoid of the loss of function mutation. According to a specific embodiment, the reduced activity is by at least 5 %, 10 %, 20 %, 30 %, 40 %, 50 %, 60 %, 70 %, 80 %, 90 % or even more as compared to that of the wild-type enzyme under the same assay conditions. Ethylene biosynthesis can be measured in small plantlets via gas chromatography (GC) or laser-based assays (Cristescu SM, Mandon J, Arslanov D, De Pessemier J, Hermans C, Harren FJM. Current methods for detecting ethylene in plants. Ann Bot-London. 2013;111(3):347-60)

According to a specific embodiment, the loss of function mutation results in no expression of the component of the ethylene biosynthesis pathway mRNA or protein (dependent on the location of the aberration in the gene encoding the component of the ethylene biosynthesis pathway).

According to a specific embodiment, the loss of function mutation results in expression of the component of the ethylene biosynthesis pathway but which is incapable or inefficient of synthesizing ethylene or a precursor thereof.

According to a specific embodiment, the loss of function mutation is selected from the group consisting of a deletion, insertion, insertion-deletion (Indel), inversion, substitution and a combination of same (e.g., deletion and substitution e.g., deletions and SNPs).

According to a specific embodiment, the loss of function mutation is smaller than 1 Kb or 0.1 Kb.

According to a specific embodiment, the "loss-of-function" mutation is in the 5' of gene encoding the component of the ethylene biosynthesis pathway so as to inhibit the production of any α expression product (e.g., exon 1).

According to a specific embodiment, the "loss-of-function" mutation is anywhere in the gene that allows the production of the expression product, while being unable to facilitate (contribute to) synthesis of ethylene or precursor thereof i.e., inactive protein. Also provided herein is a mutation in regulatory elements of the gene e.g., promoter.

As mentioned, the banana plant comprises the loss of function mutation in at least one allele of a gene encoding the component of the ethylene biosynthesis pathway.

According to a specific embodiment, the mutation is homozygous.

According to an aspect, there is provided a method of increasing shelf-life of banana, the method comprising:
(a) subjecting a banana plant cell to a DNA editing agent directed at a nucleic acid sequence encoding a component in an ethylene biosynthesis pathway of the banana to result in a loss of function mutation in the nucleic acid sequence encoding the ethylene biosynthesis pathway and
(b) regenerating a plant from the plant cell.

According to a specific embodiment, the method further comprises harvesting fruits from the plant.

According to a specific embodiment fruit is harvested still green and firm, 7-14 days prior to ripening. Each banana adult plant produces a single bunch, which is formed by many banana fruits or 'fingers' and clustered in several hands" (FAO, 2014). Banana bunches are cut by "hand" (usually involving 2-3 people) using a sharp curved knife or a machete.

As used herein "increasing shelf-life" refers to at least 10 %, 20 %, 30 %, 40 %, 50 %, 60 %, 70 %, 80 %, 85 %, 90 % or even 95 %, increase of shelf-life of harvested banana fruit having the loss of function mutation in the genome (as described herein) as compared to that of a banana plant of the same genetic background not comprising the loss of function mutation and as manifested by shelf life, as assayed by methods which are well known in the art (see Examples section which follows). Shelf-life is estimated by following the color and consistency of the fruit.

Following is a description of various non-limiting examples of methods and DNA editing agents used to introduce nucleic acid alterations to a gene of interest and agents for implementing same that can be used according to specific embodiments of the present disclosure.

Genome Editing using engineered endonucleases - this approach refers to a reverse genetics method using artificially engineered nucleases to typically cut and create specific double-stranded breaks at a desired location(s) in the genome, which are then repaired by cellular endogenous processes such as, homologous recombination (HR) or non-homologous end-joining (NHEJ). NHEJ directly joins the DNA ends in a double-stranded break, while HR utilizes a homologous donor sequence as a template (i.e. the sister chromatid formed during S-phase) for regenerating the missing DNA sequence at the break site. In order to introduce specific nucleotide modifications to the genomic DNA, a donor DNA repair template containing the desired sequence must be present during HR (exogenously provided single stranded or double stranded DNA).

Genome editing cannot be performed using traditional restriction endonucleases since most restriction enzymes recognize a few base pairs on the DNA as their target and these sequences often will be found in many locations across the genome resulting in multiple cuts which are not limited to a desired location. To overcome this challenge and create site-specific single- or double-stranded breaks, several distinct classes of nucleases have been discovered and bioengineered to date. These include the meganucleases, Zinc finger nucleases (ZFNs), transcription-activator like effector nucleases (TALENs) and CRISPR/Cas system.

*Meganucleases* - Meganucleases are commonly grouped into four families: the LAGLIDADG family, the GIY-YIG family, the His-Cys box family and the HNH family. These families are characterized by structural motifs, which affect catalytic activity and recognition sequence. For instance, members of the LAGLIDADG family are characterized by having either one or two copies of the conserved LAGLIDADG motif. The four families of meganucleases are widely separated from one another with respect to conserved structural elements and, consequently, DNA recognition sequence specificity and catalytic activity. Meganucleases are found commonly in microbial species and have the unique property of having very long recognition sequences (>14bp) thus making them naturally very specific for cutting at a desired location.

This can be exploited to make site-specific double-stranded breaks in genome editing. One of skill in the art can use these naturally occurring meganucleases, however the number of such naturally occurring meganucleases is limited. To overcome this challenge, mutagenesis and high throughput screening methods have been used to create meganuclease variants that recognize unique sequences. For example, various meganucleases have been fused to create hybrid enzymes that recognize a new sequence.

Alternatively, DNA interacting amino acids of the meganuclease can be altered to design sequence specific meganucleases (see e.g., US Patent 8,021,867). Meganucleases can be designed using the methods described in e.g., Certo, MT et al. Nature Methods (2012) 9:073-975; U.S. Patent Nos. 8,304,222; 8,021,867; 8, 119,381; 8, 124,369; 8, 129,134; 8,133,697; 8,143,015; 8,143,016; 8, 148,098; or 8, 163,514, the contents of each are incorporated herein by reference in their entirety. Alternatively, meganucleases with site specific cutting characteristics can be obtained using commercially available technologies e.g., Precision Biosciences' Directed Nuclease Editor^{™} genome editing technology.

*ZFNs and TALENs* - Two distinct classes of engineered nucleases, zinc-finger nucleases (ZFNs) and transcription activator-like effector nucleases (TALENs), have both proven to be effective at producing targeted double-stranded breaks (Christian *et al.,* 2010; Kim *et al.,* 1996; Li *et al.,* 2011; Mahfouz *et al.,* 2011; Miller *et al.,* 2010).

Basically, ZFNs and TALENs restriction endonuclease technology utilizes a non-specific DNA cutting enzyme which is linked to a specific DNA binding domain (either a series of zinc finger domains or TALE repeats, respectively). Typically a restriction enzyme whose DNA recognition site and cleaving site are separate from each other is selected. The cleaving portion is separated and then linked to a DNA binding domain, thereby yielding an endonuclease with very high specificity for a desired sequence. An exemplary restriction enzyme with such properties is FokI. Additionally FokI has the advantage of requiring dimerization to have nuclease activity and this means the specificity increases dramatically as each nuclease partner recognizes a unique DNA sequence. To enhance this effect, FokI nucleases have been engineered that can only function as heterodimers and have increased catalytic activity. The heterodimer functioning nucleases avoid the possibility of unwanted homodimer activity and thus increase specificity of the double-stranded break.

Thus, for example to target a specific site, ZFNs and TALENs are constructed as nuclease pairs, with each member of the pair designed to bind adjacent sequences at the targeted site. Upon transient expression in cells, the nucleases bind to their target sites and the FokI domains heterodimerize to create a double-stranded break. Repair of these double-stranded breaks through the non-homologous end-joining (NHEJ) pathway often results in small deletions or small sequence insertions. Since each repair made by NHEJ is unique, the use of a single nuclease pair can produce an allelic series with a range of different deletions at the target site.

In general NHEJ is relatively accurate (about 85 % of DSBs in human cells are repaired by NHEJ within about 30min from detection) in gene editing erroneous NHEJ is relied upon as when the repair is accurate the nuclease will keep cutting until the repair product is mutagenic and the recognition/cut site/PAM motif is gone/mutated or that the transiently introduced nuclease is no longer present.

The deletions typically range anywhere from a few base pairs to a few hundred base pairs in length, but larger deletions have been successfully generated in cell culture by using two pairs of nucleases simultaneously (Carlson *et al.,* 2012; Lee *et al.,* 2010). In addition, when a fragment of DNA with homology to the targeted region is introduced in conjunction with the nuclease pair, the double-stranded break can be repaired via homologous recombination (HR) to generate specific modifications (Li *et al.,* 2011; Miller *et al.,* 2010; Urnov *et al.,* 2005).

Although the nuclease portions of both ZFNs and TALENs have similar properties, the difference between these engineered nucleases is in their DNA recognition peptide. ZFNs rely on Cys2- His2 zinc fingers and TALENs on TALEs. Both of these DNA recognizing peptide domains have the characteristic that they are naturally found in combinations in their proteins. Cys2-His2 Zinc fingers are typically found in repeats that are 3 bp apart and are found in diverse combinations in a variety of nucleic acid interacting proteins. TALEs on the other hand are found in repeats with a one-to-one recognition ratio between the amino acids and the recognized nucleotide pairs. Because both zinc fingers and TALEs happen in repeated patterns, different combinations can be tried to create a wide variety of sequence specificities. Approaches for making site-specific zinc finger endonucleases include, e.g., modular assembly (where Zinc fingers correlated with a triplet sequence are attached in a row to cover the required sequence), OPEN (low-stringency selection of peptide domains vs. triplet nucleotides followed by high-stringency selections of peptide combination vs. the final target in bacterial systems), and bacterial one-hybrid screening of zinc finger libraries, among others. ZFNs can also be designed and obtained commercially from e.g., Sangamo Biosciences^{™} (Richmond, CA).

Method for designing and obtaining TALENs are described in e.g. Reyon et al. Nature Biotechnology 2012 May;30(5):460-5; Miller et al. Nat Biotechnol. (2011) 29: 143-148; Cermak et al. Nucleic Acids Research (2011) 39 (12): e82 and Zhang et al. Nature Biotechnology (2011) 29 (2): 149-53. A recently developed web-based program named Mojo Hand was introduced by Mayo Clinic for designing TAL and TALEN constructs for genome editing applications (can be accessed through www(dot)talendesign(dot)org). TALEN can also be designed and obtained commercially from e.g., Sangamo Biosciences^{™} (Richmond, CA).

*T-GEE system* (TargetGene's Genome Editing Engine) - A programmable nucleoprotein molecular complex containing a polypeptide moiety and a specificity conferring nucleic acid (SCNA) which assembles in-vivo, in a target cell, and is capable of interacting with the predetermined target nucleic acid sequence is provided. The programmable nucleoprotein molecular complex is capable of specifically modifying and/or editing a target site within the target nucleic acid sequence and/or modifying the function of the target nucleic acid sequence. Nucleoprotein composition comprises (a) polynucleotide molecule encoding a chimeric polypeptide and comprising (i) a functional domain capable of modifying the target site, and (ii) a linking domain that is capable of interacting with a specificity conferring nucleic acid, and (b) specificity conferring nucleic acid (SCNA) comprising (i) a nucleotide sequence complementary to a region of the target nucleic acid flanking the target site, and (ii) a recognition region capable of specifically attaching to the linking domain of the polypeptide. The composition enables modifying a predetermined nucleic acid sequence target precisely, reliably and cost-effectively with high specificity and binding capabilities of molecular complex to the target nucleic acid through base-pairing of specificity-conferring nucleic acid and a target nucleic acid. The composition is less genotoxic, modular in their assembly, utilize single platform without customization, practical for independent use outside of specialized core-facilities, and has shorter development time frame and reduced costs.

*CRISPR-Cas system* (also referred to herein as "CRISPR") - Many bacteria and archaea contain endogenous RNA-based adaptive immune systems that can degrade nucleic acids of invading phages and plasmids. These systems consist of clustered regularly interspaced short palindromic repeat (CRISPR) nucleotide sequences that produce RNA components and CRISPR associated (Cas) genes that encode protein components. The CRISPR RNAs (crRNAs) contain short stretches of homology to the DNA of specific viruses and plasmids and act as guides to direct Cas nucleases to degrade the complementary nucleic acids of the corresponding pathogen. Studies of the type II CRISPR/Cas system of *Streptococcus pyogenes* have shown that three components form an RNA/protein complex and together are sufficient for sequence-specific nuclease activity: the Cas9 nuclease, a crRNA containing 20 base pairs of homology to the target sequence, and a trans-activating crRNA (tracrRNA) (Jinek et al. Science (2012) 337: 816-821.).

It was further demonstrated that a synthetic chimeric guide RNA (gRNA) composed of a fusion between crRNA and tracrRNA could direct Cas9 to cleave DNA targets that are complementary to the crRNA in vitro. It was also demonstrated that transient expression of Cas9 in conjunction with synthetic gRNAs can be used to produce targeted double-stranded brakes in a variety of different species (Cho *et al.,* 2013; Cong *et al.,* 2013; DiCarlo *et al.,* 2013; Hwang *et al.,* 2013a,b; Jinek *et al.,* 2013; Mali *et al.,* 2013).

The CRIPSR/Cas system for genome editing contains two distinct components: a gRNA and an endonuclease e.g. Cas9.

The gRNA is typically a 20 nucleotide sequence encoding a combination of the target homologous sequence (crRNA) and the endogenous bacterial RNA that links the crRNA to the Cas9 nuclease (tracrRNA) in a single chimeric transcript. The gRNA/Cas9 complex is recruited to the target sequence by the base-pairing between the gRNA sequence and the complement genomic DNA. For successful binding of Cas9, the genomic target sequence must also contain the correct Protospacer Adjacent Motif (PAM) sequence immediately following the target sequence. The binding of the gRNA/Cas9 complex localizes the Cas9 to the genomic target sequence so that the Cas9 can cut both strands of the DNA causing a double-strand break. Just as with ZFNs and TALENs, the double-stranded breaks produced by CRISPR/Cas can be repaired by HR (homologous recombination) or NHEJ (non-homologous end-joining) and are susceptible to specific sequence modification during DNA repair.

The Cas9 nuclease has two functional domains: RuvC and HNH, each cutting a different DNA strand. When both of these domains are active, the Cas9 causes double strand breaks in the genomic DNA.

A significant advantage of CRISPR/Cas is that the high efficiency of this system coupled with the ability to easily create synthetic gRNAs. This creates a system that can be readily modified to target modifications at different genomic sites and/or to target different modifications at the same site. Additionally, protocols have been established which enable simultaneous targeting of multiple genes. The majority of cells carrying the mutation present biallelic mutations in the targeted genes.

However, apparent flexibility in the base-pairing interactions between the gRNA sequence and the genomic DNA target sequence allows imperfect matches to the target sequence to be cut by Cas9.

Modified versions of the Cas9 enzyme containing a single inactive catalytic domain, either RuvC- or HNH-, are called 'nickases'. With only one active nuclease domain, the Cas9 nickase cuts only one strand of the target DNA, creating a single-strand break or 'nick'. A single-strand break, or nick, is mostly repaired by single strand break repair mechanism involving proteins such as but not only, PARP (sensor) and XRCC1/LIG III complex (ligation). If a single strand break (SSB) is generated by topoisomerase I poisons or by drugs that trap PARP1 on naturally occurring SSBs then these could persist and when the cell enters into S-phase and the replication fork encounter such SSBs they will become single ended DSBs which can only be repaired by HR. However, two proximal, opposite strand nicks introduced by a Cas9 nickase are treated as a double-strand break, in what is often referred to as a 'double nick' CRISPR system. A double-nick which is basically non-parallel DSB can be repaired like other DSBs by HR or NHEJ depending on the desired effect on the gene target and the presence of a donor sequence and the cell cycle stage (HR is of much lower abundance and can only occur in S and G2 stages of the cell cycle).. Thus, if specificity and reduced off-target effects are crucial, using the Cas9 nickase to create a double-nick by designing two gRNAs with target sequences in close proximity and on opposite strands of the genomic DNA would decrease off-target effect as either gRNA alone will result in nicks that are not likely to change the genomic DNA, even though these events are not impossible.

Modified versions of the Cas9 enzyme containing two inactive catalytic domains (dead Cas9, or dCas9) have no nuclease activity while still able to bind to DNA based on gRNA specificity. The dCas9 can be utilized as a platform for DNA transcriptional regulators to activate or repress gene expression by fusing the inactive enzyme to known regulatory domains. For example, the binding of dCas9 alone to a target sequence in genomic DNA can interfere with gene transcription.

There are a number of publically available tools available to help choose and/or design target sequences as well as lists of bioinformatically determined unique gRNAs for different genes in different species such as the Feng Zhang lab's Target Finder, the Michael Boutros lab's Target Finder (E-CRISP), the RGEN Tools: Cas-OFFinder, the CasFinder: Flexible algorithm for identifying specific Cas9 targets in genomes and the CRISPR Optimal Target Finder.

Non-limiting examples of a gRNA that can be used in the present disclosure include those described in the Example section which follows.

In order to use the CRISPR system, both gRNA and Cas9 should be in a target cell or delivered as a ribonucleoprotein complex. The insertion vector can contain both cassettes on a single plasmid or the cassettes are expressed from two separate plasmids. CRISPR plasmids are commercially available such as the px330 plasmid from Addgene. Use of clustered regularly interspaced short palindromic repeats (CRISPR)-associated (Cas)-guide RNA technology and a Cas endonuclease for modifying plant genomes are also at least disclosed by Svitashev *et al.,* 2015, Plant Physiology, 169 (2): 931-945; Kumar and Jain, 2015, J Exp Bot 66: 47-57; and in U.S. Patent Application Publication No. 20150082478, which is specifically incorporated herein by reference in its entirety.

"Hit and run" or "in-out" - involves a two-step recombination procedure. In the first step, an insertion-type vector containing a dual positive/negative selectable marker cassette is used to introduce the desired sequence alteration. The insertion vector contains a single continuous region of homology to the targeted locus and is modified to carry the mutation of interest. This targeting construct is linearized with a restriction enzyme at a one site within the region of homology, introduced into the cells, and positive selection is performed to isolate homologous recombination events. The DNA carrying the homologous sequence can be provided as a plasmid, single or double stranded oligo. These homologous recombinants contain a local duplication that is separated by intervening vector sequence, including the selection cassette. In the second step, targeted clones are subjected to negative selection to identify cells that have lost the selection cassette via intrachromosomal recombination between the duplicated sequences. The local recombination event removes the duplication and, depending on the site of recombination, the allele either retains the introduced mutation or reverts to wild type. The end result is the introduction of the desired modification without the retention of any exogenous sequences.

The "double-replacement" or "tag and exchange" strategy - involves a two-step selection procedure similar to the hit and run approach, but requires the use of two different targeting constructs. In the first step, a standard targeting vector with 3' and 5' homology arms is used to insert a dual positive/negative selectable cassette near the location where the mutation is to be introduced. After the system components have been introduced to the cell and positive selection applied, HR events could be identified. Next, a second targeting vector that contains a region of homology with the desired mutation is introduced into targeted clones, and negative selection is applied to remove the selection cassette and introduce the mutation. The final allele contains the desired mutation while eliminating unwanted exogenous sequences.

Site-Specific Recombinases - The Cre recombinase derived from the P1 bacteriophage and Flp recombinase derived from the yeast *Saccharomyces cerevisiae are* site-specific DNA recombinases each recognizing a unique 34 base pair DNA sequence (termed "Lox" and "FRT", respectively) and sequences that are flanked with either Lox sites or FRT sites can be readily removed via site-specific recombination upon expression of Cre or Flp recombinase, respectively. For example, the Lox sequence is composed of an asymmetric eight base pair spacer region flanked by 13 base pair inverted repeats. Cre recombines the 34 base pair lox DNA sequence by binding to the 13 base pair inverted repeats and catalyzing strand cleavage and religation within the spacer region. The staggered DNA cuts made by Cre in the spacer region are separated by 6 base pairs to give an overlap region that acts as a homology sensor to ensure that only recombination sites having the same overlap region recombine.

Basically, the site specific recombinase system offers means for the removal of selection cassettes after homologous recombination events. This system also allows for the generation of conditional altered alleles that can be inactivated or activated in a temporal or tissue-specific manner. Of note, the Cre and Flp recombinases leave behind a Lox or FRT "scar" of 34 base pairs. The Lox or FRT sites that remain are typically left behind in an intron or 3' UTR of the modified locus, and current evidence suggests that these sites usually do not interfere significantly with gene function.

Thus, Cre/Lox and Flp/FRT recombination involves introduction of a targeting vector with 3' and 5' homology arms containing the mutation of interest, two Lox or FRT sequences and typically a selectable cassette placed between the two Lox or FRT sequences. Positive selection is applied and homologous recombination events that contain targeted mutation are identified. Transient expression of Cre or Flp in conjunction with negative selection results in the excision of the selection cassette and selects for cells where the cassette has been lost. The final targeted allele contains the Lox or FRT scar of exogenous sequences.

According to a specific embodiment, the DNA editing agent is CRISPR-Cas9. Exemplary gRNA sequences are provided herein.
>Ma04_g31490
   GACTCTAAGATCAGGGTTAAAGG (SEQ ID NO: 45);
>Ma09_g19150/Ma04_g35640/Ma04_g31490
   GCAGCTAACATCAGGGTTAAAGG (SEQ ID NO: 46).

According to a specific embodiment, the component in said ethylene biosynthesis pathway is selected from the group consisting of Ma09_g19150 (SEQ ID NO: 13), Ma04_g35640 (SEQ ID NO: 9), Ma04_g31490 (SEQ ID NO: 8), Ma01_g11540 (SEQ ID NO: 20) and Ma07_g19730 (SEQ ID NO: 27).

According to a specific embodiment, the component in said ethylene biosynthesis pathway is selected from the group consisting of Ma04_g35640 (SEQ ID NO: 9) and Ma07_g19730 (SEQ ID NO: 27).

According to a specific embodiment, the component in said ethylene biosynthesis pathway is selected from the group consisting of Ma09_g19150 (SEQ ID NO: 13), Ma04_g31490 (SEQ ID NO: 8) and Ma01_g11540 (SEQ ID NO: 20).

According to a specific embodiment, the DNA editing agent is directed at nucleic acid coordinates which specifically target more than one nucleic acid sequence encoding said component in said ethylene biosynthesis pathway.

According to a specific embodiment, the DNA editing agent comprises a nucleic acid sequence at least 99 % identical to a nucleic acid sequence selected from the group consisting of SEQ ID NO: 47-54 (sgRNAs: 183, 184, 188, 189, 190, 191, 194 and 195).

According to a specific embodiment, the DNA editing agent comprises a nucleic acid sequence at least 99 % identical to a nucleic acid sequence set forth in SEQ ID NO: 47 (sgRNA: 183).

According to a specific embodiment, the DNA editing agent comprises a nucleic acid set forth in SEQ ID NO: 47 (sgRNA: 183).

According to a specific embodiment, the DNA editing agent comprises a plurality of nucleic acid sequences set forth in SEQ ID NO: 47-54 (sgRNAs: 183, 184, 188, 189, 190, 191, 194 and 195)

According to a specific embodiment, the DNA editing agent comprises a plurality of nucleic acid sequences set forth in SEQ ID NO: 47, 49 and/or 50 (sgRNAs: 183, 188, 189).

According to a specific embodiment, the DNA editing agent comprises a plurality of nucleic acid sequences set forth in SEQ ID NO: 51 and/or 53 (sgRNAs: 190 and 194).

The DNA editing agent is typically introduced into the plant cell using expression vectors.

Thus, according to an aspect of the invention there is provided a nucleic acid construct comprising a nucleic acid sequence coding for a DNA editing agent capable of hybridizing to a gene encoding a component of the biosynthesis of ethylene of a banana and facilitating editing of said gene, said nucleic acid sequence being operably linked to a cis-acting regulatory element for expressing said DNA editing agent in a cell of a banana.

Embodiments of the invention relate to any DNA editing agent, such as described above.

According to a specific embodiment, the genome editing agent comprises an endonuclease, which may comprise or have an auxiliary unit of a DNA targeting module (e.g., sgRNA, or also as referred to herein as "gRNA").

According to a specific embodiment, the DNA editing agent is CRISPR/Cas9 sgRNA.

According to a specific embodiment, the nucleic acid construct further comprises a nucleic acid sequence encoding an endonuclease of a DNA editing agent (e.g., Cas9 or the endonucleases described above).

According to another specific embodiment, the endonuclease and the sgRNA are encoded from different constructs whereby each is operably linked to a cis-acting regulatory element active in plant cells (e.g., promoter).

In a particular embodiment of some embodiments of the invention the regulatory sequence is a plant-expressible promoter.

Constructs useful in the methods according to some embodiments may be constructed using recombinant DNA technology well known to persons skilled in the art. Such constructs may be commercially available, suitable for transforming into plants and suitable for expression of the gene of interest in the transformed cells.

As used herein the phrase "plant-expressible" refers to a promoter sequence, including any additional regulatory elements added thereto or contained therein, is at least capable of inducing, conferring, activating or enhancing expression in a plant cell, tissue or organ, preferably a monocotyledonous or dicotyledonous plant cell, tissue, or organ. Examples of promoters useful for the methods of some embodiments of the invention include, but are not limited to, Actin, CANV 35S, CaMV19S, GOS2. Promoters which are active in various tissues, or developmental stages can also be used.

Nucleic acid sequences of the polypeptides of some embodiments of the invention may be optimized for plant expression. Examples of such sequence modifications include, but are not limited to, an altered G/C content to more closely approach that typically found in the plant species of interest, and the removal of codons atypically found in the plant species commonly referred to as codon optimization.

Plant cells may be transformed stably or transiently with the nucleic acid constructs of some embodiments of the invention. In stable transformation, the nucleic acid molecule of some embodiments of the invention is integrated into the plant genome and as such it represents a stable and inherited trait. In transient transformation, the nucleic acid molecule is expressed by the cell transformed but it is not integrated into the genome and as such it represents a transient trait.

According to a specific embodiment, the plant is transiently transfected with a DNA editing agent.

According to a specific embodiment, promoters in the nucleic acid construct comprise a Pol3 promoter. Examples of Pol3 promoters include, but are not limited to, AtU6-29, AtU626, AtU3B, AtU3d, TaU6.

According to a specific embodiment, promoters in the nucleic acid construct comprise a Pol2 promoter. Examples of Pol2 promoters include, but are not limited to, CaMV 35S, CaMV 19S, ubiquitin, CVMV.

According to a specific embodiment, promoters in the nucleic acid construct comprise a 35S promoter.

According to a specific embodiment, promoters in the nucleic acid construct comprise a U6 promoter.

According to a specific embodiment, promoters in the nucleic acid construct comprise a Pol 3 (e.g., U6) promoter operatively linked to the nucleic acid agent encoding at least one gRNA and/or a Pol2 (e.g., CamV35S) promoter operatively linked to the nucleic acid sequence encoding the genome editing agent or the nucleic acid sequence encoding the fluorescent reporter (as described in a specific embodiment below).

According to a specific embodiment, the construct is useful for transient expression by Agrobacterium-mediated transformation (Helens et al., 2005, Plant Methods 1:13). Methods of transient transformation are further described herein.

According to a specific embodiment, the nucleic acid sequences comprised in the construct are devoid of sequences which are homologous to the plant cell's genome other than any guide sequences in sgRNA sequences so as to avoid integration to the plant genome.

In certain embodiments, the nucleic acid construct is a non-integrating construct, preferably where the nucleic acid sequence encoding the fluorescent reporter is also non-integrating. As used herein, "non-integrating" refers to a construct or sequence that is not affirmatively designed to facilitate integration of the construct or sequence into the genome of the plant of interest. For example, a functional T-DNA vector system for *Agrobacterium-*mediated genetic transformation is not a non-integrating vector system as the system is affirmatively designed to integrate into the plant genome. Similarly, a fluorescent reporter gene sequence or selectable marker sequence that has flanking sequences that are homologous to the genome of the plant of interest to facilitate homologous recombination of the fluorescent reporter gene sequence or selectable marker sequence into the genome of the plant of interest would not be a non-integrating fluorescent reporter gene sequence or selectable marker sequence.

Various cloning kits can be used according to the teachings of some embodiments of the invention.

According to a specific embodiment the nucleic acid construct is a binary vector. Examples for binary vectors are pBIN19, pBI101, pBinAR, pGPTV, pCAMBIA, pBIB-HYG, pBecks, pGreen or pPZP (Hajukiewicz, P. et al., Plant Mol. Biol. 25, 989 (1994), and Hellens et al, Trends in Plant Science 5, 446 (2000)).

Examples of other vectors to be used in other methods of DNA delivery (e.g. transfection, electroporation, bombardment, viral inoculation) are: pGE-sgRNA (Zhang et al. Nat. Comms. 2016 7:12697), pJIT163-Ubi-Cas9 (Wang et al. Nat. Biotechnol 2004 32, 947-951), pICH47742::2x35S-5'UTR-hCas9(STOP)-NOST (Belhan et al. Plant Methods 2013 11;9(1):39).

Embodiments described herein also relate to a method of selecting cells comprising a genome editing event, the method comprising:
(a) transforming cells of a banana plant with a nucleic acid construct comprising the genome editing agent (as described above) and a fluorescent reporter;
(b) selecting transformed cells exhibiting fluorescence emitted by the fluorescent reporter using flow cytometry or imaging;
(c) culturing the transformed cells comprising the genome editing event by the DNA editing agent for a time sufficient to lose expression of the DNA editing agent so as to obtain cells which comprise a genome editing event generated by the DNA editing agent but lack DNA encoding the DNA editing agent; and

According to some embodiments, the method further comprises validating in the transformed cells, loss of expression of the fluorescent reporter following step (c).

According to some embodiments, the method further comprises validating in the transformed cells loss, of expression of the DNA editing agent following step (c).

A non-limiting embodiment of the method is described in the Flowchart of Figure 1.

According to a specific embodiment, the plant is a plant cell e.g., plant cell in an embryonic cell suspension.

According to a specific embodiment, the plant cell is a protoplast.

The protoplasts are derived from any plant tissue e.g., roots, leaves, embryonic cell suspension, calli or seedling tissue.

There are a number of methods of introducing DNA into plant cells e.g., using protoplasts and the skilled artisan will know which to select.

The delivery of nucleic acids may be introduced into a plant cell in embodiments of the invention by any method known to those of skill in the art, including, for example and without limitation: by transformation of protoplasts (See, e.g., U.S. Pat. No. 5,508,184); by desiccation/inhibition-mediated DNA uptake (See, e.g., Potrykus et al. (1985) Mol. Gen. Genet. 199:183-8); by electroporation (See, e.g., U.S. Pat. No. 5,384,253); by agitation with silicon carbide fibers (See, e.g., U.S. Pat. Nos. 5,302,523 and 5,464,765); by Agrobacterium-mediated transformation (See, e.g., U.S. Pat. Nos. 5,563,055, 5,591,616, 5,693,512, 5,824,877, 5,981,840, and 6,384,301); by acceleration of DNA-coated particles (See, e.g., U.S. Pat. Nos. 5,015,580, 5,550,318, 5,538,880, 6,160,208, 6,399,861, and 6,403,865) and by Nanoparticles, nanocarriers and cell penetrating peptides (WO201126644A2; WO2009046384A1; WO2008148223A1) in the methods to deliver DNA, RNA, Peptides and/or proteins or combinations of nucleic acids and peptides into plant cells.

Other methods of transfection include the use of transfection reagents (e.g. Lipofectin, ThermoFisher), dendrimers (Kukowska-Latallo, J.F. et al., 1996, Proc. Natl. Acad. Sci. USA93, 4897-902), cell penetrating peptides (Mäe et al., 2005, Internalisation of cell-penetrating peptides into tobacco protoplasts, Biochimica et Biophysica Acta 1669(2): 101-7) or polyamines (Zhang and Vinogradov, 2010, Short biodegradable polyamines for gene delivery and transfection of brain capillary endothelial cells, J Control Release, 143(3):359-366).

According to a specific embodiment, the introduction of DNA into plant cells (e.g., protoplasts) is effected by electroporation.

According to a specific embodiment, the introduction of DNA into plant cells (e.g., protoplasts) is effected by bombardment/biolistics.

According to a specific embodiment, for introducing DNA into protoplasts the method comprises polyethylene glycol (PEG)-mediated DNA uptake. For further details see Karesch et al. (1991) Plant Cell Rep. 9:575-578; Mathur et al. (1995) Plant Cell Rep. 14:221-226; Negrutiu et al. (1987) Plant Cell Mol. Biol. 8:363-373. Protoplasts are then cultured under conditions that allowed them to grow cell walls, start dividing to form a callus, develop shoots and roots, and regenerate whole plants.

Transient transformation can also be effected by viral infection using modified plant viruses.

Viruses that have been shown to be useful for the transformation of plant hosts include CaMV, TMV, TRV and BV. Transformation of plants using plant viruses is described in U.S. Pat. No. 4,855,237 (BGV), EP-A 67,553 (TMV), Japanese Published Application No. 63-14693 (TMV), EPA 194,809 (BV), EPA 278,667 (BV); and Gluzman, Y. et al., Communications in Molecular Biology: Viral Vectors, Cold Spring Harbor Laboratory, New York, pp. 172-189 (1988). Pseudovirus particles for use in expressing foreign DNA in many hosts, including plants, is described in WO 87/06261.

Construction of plant RNA viruses for the introduction and expression of non-viral exogenous nucleic acid sequences in plants is demonstrated by the above references as well as by Dawson, W. O. et al., Virology (1989) 172:285-292; Takamatsu et al. EMBO J. (1987) 6:307-311; French et al. Science (1986) 231:1294-1297; and Takamatsu et al. FEBS Letters (1990) 269:73-76.

When the virus is a DNA virus, suitable modifications can be made to the virus itself. Alternatively, the virus DNA can first be cloned into a bacterial plasmid for ease of constructing the desired viral vector with the foreign DNA. The virus DNA can then be excised from the plasmid. If the virus is a DNA virus, a bacterial origin of replication can be attached to the viral DNA, which is then replicated by the bacteria. Transcription and translation of this DNA will produce the coat protein which will encapsidate the viral DNA. If the virus is an RNA virus, the virus is generally cloned as a cDNA and inserted into a plasmid. The plasmid is then used to make all of the constructions. The RNA virus is then produced by transcribing the viral sequence of the plasmid and translation of the viral genes to produce the coat protein(s) which encapsidate the viral RNA.

Construction of plant RNA viruses for the introduction and expression in plants of non-viral exogenous nucleic acid sequences such as those included in the construct of some embodiments of the invention is demonstrated by the above references as well as in U.S. Pat. No. 5,316,931.

In one embodiment, a plant viral nucleic acid is provided in which the native coat protein coding sequence has been deleted from a viral nucleic acid, a non-native plant viral coat protein coding sequence and a non-native promoter, preferably the subgenomic promoter of the non-native coat protein coding sequence, capable of expression in the plant host, packaging of the recombinant plant viral nucleic acid, and ensuring a systemic infection of the host by the recombinant plant viral nucleic acid, has been inserted. Alternatively, the coat protein gene may be inactivated by insertion of the non-native nucleic acid sequence within it, such that a protein is produced. The recombinant plant viral nucleic acid may contain one or more additional non-native subgenomic promoters. Each non-native subgenomic promoter is capable of transcribing or expressing adjacent genes or nucleic acid sequences in the plant host and incapable of recombination with each other and with native subgenomic promoters. Non-native (foreign) nucleic acid sequences may be inserted adjacent the native plant viral subgenomic promoter or the native and a non-native plant viral subgenomic promoters if more than one nucleic acid sequence is included. The non-native nucleic acid sequences are transcribed or expressed in the host plant under control of the subgenomic promoter to produce the desired products.

In a second embodiment, a recombinant plant viral nucleic acid is provided as in the first embodiment except that the native coat protein coding sequence is placed adjacent one of the non-native coat protein subgenomic promoters instead of a non-native coat protein coding sequence.

In a third embodiment, a recombinant plant viral nucleic acid is provided in which the native coat protein gene is adjacent its subgenomic promoter and one or more non-native subgenomic promoters have been inserted into the viral nucleic acid. The inserted non-native subgenomic promoters are capable of transcribing or expressing adjacent genes in a plant host and are incapable of recombination with each other and with native subgenomic promoters. Non-native nucleic acid sequences may be inserted adjacent the non-native subgenomic plant viral promoters such that said sequences are transcribed or expressed in the host plant under control of the subgenomic promoters to produce the desired product.

In a fourth embodiment, a recombinant plant viral nucleic acid is provided as in the third embodiment except that the native coat protein coding sequence is replaced by a non-native coat protein coding sequence.

The viral vectors are encapsidated by the coat proteins encoded by the recombinant plant viral nucleic acid to produce a recombinant plant virus. The recombinant plant viral nucleic acid or recombinant plant virus is used to infect appropriate host plants. The recombinant plant viral nucleic acid is capable of replication in the host, systemic spread in the host, and transcription or expression of foreign gene(s) (isolated nucleic acid) in the host to produce the desired protein.

Regardless of the transformation/infection method employed, the present teachings further relate to any cell e.g., a plant cell (e.g., protoplast) or a bacterial cell comprising the nucleic acid construct(s) as described herein.

Following transformation, cells are subjected to flow cytometry to select transformed cells exhibiting fluorescence emitted by the fluorescent reporter (i.e., fluorescent protein").

As used herein, "a fluorescent protein" refers to a polypeptide that emits fluorescence and is typically detectable by flow cytometry or imaging, therefore can be used as a basis for selection of cells expressing such a protein.

Examples of fluorescent proteins that can be used as reporters are the Green Fluorescent Protein (GFP), the Blue Fluorescent Protein (BFP) and the red fluorescent protein dsRed. A non-limiting list of fluorescent or other reporters includes proteins detectable by luminescence (e.g. luciferase) or colorimetric assay (e.g. GUS). According to a specific embodiment, the fluorescent reporter is DsRed or GFP.

This analysis is typically effected within 24-72 hours e.g., 48-72, 24-28 hours, following transformation. To ensure transient expression, no antibiotic selection is employed e.g., antibiotics for a selection marker. The culture may still comprise antibiotics but not to a selection marker.

Flow cytometry of plant cells is typically performed by Fluorescence Activated Cell Sorting (FACS). Fluorescence activated cell sorting (FACS) is a well-known method for separating particles, including cells, based on the fluorescent properties of the particles (see, e.g., Kamarch, 1987, Methods Enzymol, 151:150-165).

For instance, FACS of GFP-positive cells makes use of the visualization of the green versus the red emission spectra of protoplasts excited by a 488 nm laser. GFP-positive protoplasts can be distinguished by their increased ratio of green to red emission.

Following is a non-binding protocol adapted from Bastiaan et al. J Vis Exp. 2010; (36): 1673, which is hereby incorporated by reference. FACS apparati are commercially available e.g., FACSMelody (BD), FACSAria (BD).

A flow stream is set up with a 100 µm nozzle and a 20 psi sheath pressure. The cell density and sample injection speed can be adjusted to the particular experiment based on whether a best possible yield or fastest achievable speed is desired, e.g., up to 10,000,000 cells/ml. The sample is agitated on the FACS to prevent sedimentation of the protoplasts. If clogging of the FACS is an issue, there are three possible troubleshooting steps: 1. Perform a sample-line backflush. 2. Dilute protoplast suspension to reduce the density. 3. Clean up the protoplast solution by repeating the filtration step after centrifugation and resuspension. The apparatus is prepared to measure forward scatter (FSC), side scatter (SSC) and emission at 530/30 nm for GFP and 610/20 nm for red spectrum auto-fluorescence (RSA) after excitation by a 488 nm laser. These are in essence the only parameters used to isolate GFP-positive protoplasts. The voltage settings can be used: FSC - 60V, SSC 250V, GFP 350V and RSA 335V. Note that the optimal voltage settings will be different for every FACS and will even need to be adjusted throughout the lifetime of the cell sorter.

The process is started by setting up a dotplot for forward scatter versus side scatter. The voltage settings are applied so that the measured events are centered in the plot. Next, a dot plot is created of green versus red fluorescence signals. The voltage settings are applied so that the measured events yield a centered diagonal population in the plot when looking at a wild-type (non-GFP) protoplast suspension. A protoplast suspension derived from a GFP marker line will produce a clear population of green fluorescent events never seen in wild-type samples. Compensation constraints are set to adjust for spectral overlap between GFP and RSA. Proper compensation constraint settings will allow for better separation of the GFP-positive protoplasts from the non-GFP protoplasts and debris. The constraints used here are as follows: RSA, minus 17.91% GFP. A gate is set to identify GFP-positive events, a negative control of non-GFP protoplasts should be used to aid in defining the gate boundaries. A forward scatter cutoff is implemented in order to leave small debris out of the analysis. The GFP-positive events are visualized in the FSC vs. SSC plot to help determine the placement of the cutoff. E.g., cutoff is set at 5,000. Note that the FACS will count debris as sort events and a sample with high levels of debris may have a different percent GFP positive events than expected. This is not necessarily a problem. However, the more debris in the sample, the longer the sort will take. Depending on the experiment and the abundance of the cell type to be analyzed, the FACS precision mode is set either for optimal yield or optimal purity of the sorted cells.

Following FACS sorting, positively selected pools of transformed plant cells, (e.g., protoplasts) displaying the fluorescent marker are collected and an aliquot can be used for testing the DNA editing event (optional step, see Figure 1). Alternatively (or following optional validating) the clones are cultivated in the absence of selection (e.g., antibiotics for a selection marker) until they develop into colonies i.e., clones (at least 28 days) and micro-calli. Following at least 60-100 days in culture (e.g., at least 70 days, at least 80 days), a portion of the cells of the calli are analyzed (validated) for: the DNA editing event and the presence of the DNA editing agent, namely, loss of DNA sequences encoding for the DNA editing agent, pointing to the transient nature of the method.

Thus, clones are validated for the presence of a DNA editing event also referred to herein as "mutation" or "edit", dependent on the type of editing sought e.g., insertion, deletion, insertion-deletion (Indel), inversion, substitution and combinations thereof.

According to a specific embodiment, the genome editing event comprises a deletion, a single base pair substitution, or an insertion of genetic material from a second plant that could otherwise be introduced into the plant of interest by traditional breeding.

According to a specific embodiment, the genome editing event does not comprise an introduction of foreign DNA into a genome of the plant of interest that could not be introduced through traditional breeding.

Methods for detecting sequence alteration are well known in the art and include, but not limited to, DNA sequencing (e.g., next generation sequencing), electrophoresis, an enzyme-based mismatch detection assay and a hybridization assay such as PCR, RT-PCR, RNase protection, in-situ hybridization, primer extension, Southern blot, Northern Blot and dot blot analysis. Various methods used for detection of single nucleotide polymorphisms (SNPs) can also be used, such as PCR based T7 endonuclease, Heteroduplex and Sanger sequencing.

Another method of validating the presence of a DNA editing event e.g., Indels comprises a mismatch cleavage assay that makes use of a structure selective enzyme (e.g. endonuclease) that recognizes and cleaves mismatched DNA.

The mismatch cleavage assay is a simple and cost-effective method for the detection of indels and is therefore the typical procedure to detect mutations induced by genome editing. The assay uses enzymes that cleave heteroduplex DNA at mismatches and extrahelical loops formed by multiple nucleotides, yielding two or more smaller fragments. A PCR product of ~ 300-1000 bp is generated with the predicted nuclease cleavage site off-center so that the resulting fragments are dissimilar in size and can easily be resolved by conventional gel electrophoresis or high-performance liquid chromatography (HPLC). End-labeled digestion products can also be analyzed by automated gel or capillary electrophoresis. The frequency of indels at the locus can be estimated by measuring the integrated intensities of the PCR amplicon and cleaved DNA bands. The digestion step takes 15-60 min, and when the DNA preparation and PCR steps are added the entire assays can be completed in < 3 h.

Two alternative enzymes are typically used in this assay. T7 endonuclease 1 (T7E1) is a resolvase that recognizes and cleaves imperfectly matched DNA at the first, second or third phosphodiester bond upstream of the mismatch. The sensitivity of a T7E1-based assay is 0.5-5 %. In contrast, Surveyor^{™} nuclease (Transgenomic Inc., Omaha, NE, USA) is a member of the CEL family of mismatch-specific nucleases derived from celery. It recognizes and cleaves mismatches due to the presence of single nucleotide polymorphisms (SNPs) or small indels, cleaving both DNA strands downstream of the mismatch. It can detect indels of up to 12 nt and is sensitive to mutations present at frequencies as low as ~ 3%, *i.e.* 1 in 32 copies.

Yet another method of validating the presence of an editing even comprises the high-resolution melting analysis.

High-resolution melting analysis (HRMA) involves the amplification of a DNA sequence spanning the genomic target (90-200 bp) by real-time PCR with the incorporation of a fluorescent dye, followed by melt curve analysis of the amplicons. HRMA is based on the loss of fluorescence when intercalating dyes are released from double-stranded DNA during thermal denaturation. It records the temperature-dependent denaturation profile of amplicons and detects whether the melting process involves one or more molecular species.

Yet another method is the heteroduplex mobility assay. Mutations can also be detected by analyzing re-hybridized PCR fragments directly by native polyacrylamide gel electrophoresis (PAGE). This method takes advantage of the differential migration of heteroduplex and homoduplex DNA in polyacrylamide gels. The angle between matched and mismatched DNA strands caused by an indel means that heteroduplex DNA migrates at a significantly slower rate than homoduplex DNA under native conditions, and they can easily be distinguished based on their mobility. Fragments of 140-170 bp can be separated in a 15% polyacrylamide gel. The sensitivity of such assays can approach 0.5% under optimal conditions, which is similar to T7E1 (. After reannealing the PCR products, the electrophoresis component of the assay takes ~ 2 h.

Other methods of validating the presence of editing events are described in length in Zischewski 2017 Biotechnol. Advances 1(1):95-104.

It will be appreciated that positive clones can be homozygous or heterozygous for the DNA editing event. The skilled artisan will select the clone for further culturing/regeneration according to the intended use.

Clones exhibiting the presence of a DNA editing event as desired are further analyzed for the presence of the DNA editing agent. Namely, loss of DNA sequences encoding for the DNA editing agent, pointing to the transient nature of the method.

This can be done by analyzing the expression of the DNA editing agent (e.g., at the mRNA, protein) e.g., by fluorescent detection of GFP or q-PCR.,

Alternatively, or additionally, the cells are analyzed for the presence of the nucleic acid construct as described herein or portions thereof e.g., nucleic acid sequence encoding the reporter polypeptide or the DNA editing agent.

Clones showing no DNA encoding the fluorescent reporter or DNA editing agent (e.g., as affirmed by fluorescent microscopy, q-PCR and or any other method such as Southern blot, PCR, sequencing) yet comprising the DNA editing event(s) [mutation(s)] as desired are isolated for further processing.

These clones can therefore be stored (e.g., cryopreserved).

Alternatively, cells (e.g., protoplasts) may be regenerated into whole plants first by growing into a group of plant cells that develops into a callus and then by regeneration of shoots (caulogenesis) from the callus using plant tissue culture methods. Growth of protoplasts into callus and regeneration of shoots requires the proper balance of plant growth regulators in the tissue culture medium that must be customized for each species of plant

Protoplasts may also be used for plant breeding, using a technique called protoplast fusion. Protoplasts from different species are induced to fuse by using an electric field or a solution of polyethylene glycol. This technique may be used to generate somatic hybrids in tissue culture.

Methods of protoplast regeneration are well known in the art. Several factors affect the isolation, culture, and regeneration of protoplasts, namely the genotype, the donor tissue and its pre-treatment, the enzyme treatment for protoplast isolation, the method of protoplast culture, the culture, the culture medium, and the physical environment. For a thorough review see Maheshwari et al. 1986 Differentiation of Protoplasts and of Transformed Plant Cells: 3-36. Springer-Verlag, Berlin.

The regenerated plants can be subjected to further breeding and selection as the skilled artisan sees fit.

The plant or cells thereof are devoid of a transgene encoding a DNA editing agent.

The phenotype of the final lines, plants or intermediate breeding products can be analyzed such as by determining the sequence of gene encoding the component of the ethylene biosynthesis pathway, expression thereof in the mRNA or protein level, activity of the protein and/or analyzing the properties of the fruit (shelf-life).
Ethylene production: Ethylene biosynthesis can be measured in small plantlets via gas chromatography (GC) or laser-based assays (Cristescu SM, Mandon J, Arslanov D, De Pessemier J, Hermans C, Harren FJM. Current methods for detecting ethylene in plants. Ann Bot-London. 2013;111(3):347-60).

As is illustrated herein and in the Examples section which follows. The present inventors were able to transform banana with a genome editing agent(s), while avoiding stable transgenesis.

Hence the present methodology allows genome editing without integration of a selectable or screenable reporter.

Thus, embodiments of the invention further relate to plants, plant cells and processed product of plants comprising the gene editing event(s) generated according to the present teachings,

Thus, the present teachings also relate to parts of the plants as described herein or processed products thereof.

Banana fruit, and banana fruit based products as well as their methods of producing are contemplated using the plants described herein.

Also contemplated are banana-by-products and methods of producing same such as peels, leaves, pseudostem, stalk and inflorescence in various food and non-food applications serving as thickening agent, coloring and flavor, alternative source for macro and micronutrients, nutraceuticals, livestock feed, natural fibers, and sources of natural bioactive compounds and bio-fertilizers.

According to a specific-embodiment, processed products comprise DNA.

It is expected that during the life of a patent maturing from this application many relevant DNA editing agents will be developed and the scope of the term DNA editing agent is intended to include all such new technologies *a priori.*

As used herein the term "about" refers to ± 10 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

When reference is made to particular sequence listings, such reference is to be understood to also encompass sequences that substantially correspond to its complementary sequence as including minor sequence variations, resulting from, e.g., sequencing errors, cloning errors, or other alterations resulting in base substitution, base deletion or base addition, provided that the frequency of such variations is less than 1 in 50 nucleotides, alternatively, less than 1 in 100 nucleotides, alternatively, less than 1 in 200 nucleotides, alternatively, less than 1 in 500 nucleotides, alternatively, less than 1 in 1000 nucleotides, alternatively, less than 1 in 5,000 nucleotides, alternatively, less than 1 in 10,000 nucleotides.

It is understood that any Sequence Identification Number (SEQ ID NO) disclosed in the instant application can refer to either a DNA sequence or a RNA sequence, depending on the context where that SEQ ID NO is mentioned, even if that SEQ ID NO is expressed only in a DNA sequence format or a RNA sequence format. For example, a given SEQ ID NO: is expressed in a DNA sequence format (*e.g.,* reciting T for thymine), but it can refer to either a DNA sequence that corresponds to a given nucleic acid sequence, or the RNA sequence of an RNA molecule nucleic acid sequence. Similarly, though some sequences are expressed in a RNA sequence format (*e.g.,* reciting U for uracil), depending on the actual type of molecule being described, it can refer to either the sequence of a RNA molecule comprising a dsRNA, or the sequence of a DNA molecule that corresponds to the RNA sequence shown. In any event, both DNA and RNA molecules having the sequences disclosed with any substitutes are envisioned.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

As used herein the term "about" refers to ± 10 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

When reference is made to particular sequence listings, such reference is to be understood to also encompass sequences that substantially correspond to its complementary sequence as including minor sequence variations, resulting from, e.g., sequencing errors, cloning errors, or other alterations resulting in base substitution, base deletion or base addition, provided that the frequency of such variations is less than 1 in 50 nucleotides, alternatively, less than 1 in 100 nucleotides, alternatively, less than 1 in 200 nucleotides, alternatively, less than 1 in 500 nucleotides, alternatively, less than 1 in 1000 nucleotides, alternatively, less than 1 in 5,000 nucleotides, alternatively, less than 1 in 10,000 nucleotides.

It is understood that any Sequence Identification Number (SEQ ID NO) disclosed in the instant application can refer to either a DNA sequence or a RNA sequence, depending on the context where that SEQ ID NO is mentioned, even if that SEQ ID NO is expressed only in a DNA sequence format or a RNA sequence format. For example, a SEQ ID NO: is expressed in a DNA sequence format (*e.g.,* reciting T for thymine), but it can refer to either a DNA sequence that corresponds to a nucleic acid sequence, or the RNA sequence of an RNA molecule nucleic acid sequence. Similarly, though some sequences are expressed in a RNA sequence format (*e.g.,* reciting U for uracil), depending on the actual type of molecule being described, it can refer to either the sequence of a RNA molecule comprising a dsRNA, or the sequence of a DNA molecule that corresponds to the RNA sequence shown. In any event, both DNA and RNA molecules having the sequences disclosed with any substitutes are envisioned.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non-limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### MATERIALS AND METHODS

### Embryogenic callus and cell suspension generation and maintenance.

An embryogenic callus is developed from an initial explant such as immature male flowers or shoot tip as described by Ma, 1988 (Ma S.S. 1991 Somatic embryogenesis and plant regeneration from cell suspension culture of banana. In Proceedings of Symposium on Tissue culture of horticultural crops, Taipei, Taiwan, 8-9 March 1988, pp. 181-188) and Schoofs, 1997 (Schoofs H. 1997. The origin of embryogenic cells in Musa. PhD thesis, KULeuven, Belgium). Embryogenic cell suspensions are initiated from freshly developed highly embryogenic calli in liquid medium. 80% of the medium is refreshed every 12-14 days until the initiated cell suspension is fully established (6-9 months).

### sgRNA cloning

The transfection plasmid utilized was composed of 4 modules comprising of 1, eGFP driven by the CaMV35s promoter terminated by a G7 temination sequence; 2, Cas9 (human codon optimised) driven by the CaMV35s promoter terminated by Mas termination sequence ; 3, AtU6 promoter driving sgRNA for guide 1; 4 AtU6 promoter driving sgRNA for guide 2. A binary vector can be used such as pCAMBIA or pRI-201-AN DNA.

### Gene editing system validation by targeting exogenous reporter gene GFP

The non-transgenic GE system proposed here was validated and optimized through targeting the DNA of exogenous gene (GFP). To analyze the strength of different RNA polymerase III (pol-III) promoters sgRNA were designed for targeting eGFP in the CRISPR Cas9 complex and then the effect of different promoters in knocking out eGFP expression in transformed cells was tested.

Specifically, plasmids (e.g. pBluescript, pUC19) contained four transcriptional units containing Cas9, eGFP, dsRED, and sgRNA-GFP driven by different pol-II and pol-III promoters (e.g. CAMV 35S, U6). These plasmids were transfected into protoplast cultures and analyzed by FACS after a 24-72 hour incubation period. High frequency in dsRED (or mCherry, RFP) expression indicated high transfection efficiency, while low frequency in eGFP expression indicated successful gene editing through CRISPR-Cas9. Therefore the line that showed the lowest eGFP:dsRED expression ratio was the chosen pol-III promoter as it caused the highest proportion of eGFP inactivation through CRISPR Cas9 complexes.

### Final plasmid design

For transient expression, a plasmid containing four transcriptional units was used. The first transcriptional unit contained the CaMV-35S promoter-driving expression of Cas9 and the tobacco mosaic virus (TMV) terminator. The next transcriptional unit consisted of another CaMV-35S promoter driving expression of eGFP and the nos terminator. The third and fourth transcriptional units each contained the Arabidopsis U6 promoter expressing sgRNA to target genes (as mentioned each vector comprises two sgRNAs).

### Protoplasts isolation

Protoplasts were isolated by incubating plant material (e.g. leaves, calli, cell suspensions) in a digestion solution (1% cellulase, 0.5% macerozyme, 0.5% driselase, 0.4M mannitol, 154mM NaCl, 20mM KCl, 20mM MES pH 5.6, 10mM CaCl2) for 4-24h at room temperature and gentle shaking. After digestion, remaining plant material was washed with W5 solution (154mM NaCl, 125 mM CaCl2, 5mM KCl, 2mM MES pH5.6) and protoplasts suspension was filtered through a 40um strainer. After centrifugation at 80g for 3min at room temperature, protoplasts were resuspended in 2ml W5 buffer and precipitated by gravity in ice. The final protoplast pellet was resuspended in 2ml of MMg (0.4M mannitol, 15mM MagCl2, 4mM MES pH 5.6) and protoplast concentration was determined using a hemocytometer. Protoplasts viability was estimated using Trypan Blue staining.

**Polyethylene glycol (PEG)-mediated plasmid transfection.** PEG-transfection of banana protoplasts was effected using a modified version of the strategy reported by Wang et al,. (2015) [Wang, H., et al., An efficient PEG-mediated transient gene expression system in grape protoplasts and its application in subcellular localization studies of flavonoids biosynthesis enzymes. Scientia Horticulturae, 2015. 191: p. 82-89]. Protoplasts were resuspended to a density of 2-5 × 10⁶ protoplasts/ml in MMg solution. 100-200 µl of protoplast suspension was added to a tube containing the plasmid. The plasmid:protoplast ratio greatly affects transformation efficiency therefore a range of plasmid concentrations in protoplast suspension, 5 - 300 µg/µl, were assayed. PEG solution (100-200 µl) was added to the mixture and incubated at 23 °C for various lengths of time ranging from 10 - 60 minutes. PEG4000 concentration was optimized, a range of 20 - 80 % PEG4000 in 200-400 mM mannitol, 100-500 mM CaCl₂ solution was assayed. The protoplasts were then washed in W5 and centrifugated at 80g for 3min, prior resuspension in 1ml W5 and incubated in the dark at 23 °C. After incubation for 24-72h fluorescence was detected by microscopy.

### Electroporation

A plasmid containing Pol2-driven GFP/RFP, Pol2-driven-NLS-Cas9 and Pol3-driven sgRNA targeting the relevant genes (see list of Table 2 above) was introduced to the cells using electroporation (BIORAD-GenePulserII; Miao and Jian 2007 Nature Protocols 2(10): 2348-2353. 500 µl of protoplasts were transferred into electroporation cuvettes and mixed with 100 µl of plasmid (10-40 µg DNA). Protoplasts were electroporated at 130 V and 1,000 F and incubated at room temperature for 30 minutes. 1 ml of protoplast culture medium was added to each cuvette and the protoplast suspension was poured into a small petri dish. After incubation for 24-48h fluorescence was detected by microscopy.

### FACS sorting of fluorescent protein-expressing cells

48 hrs after plasmid/RNA delivery, cells were collected and sorted for fluorescent protein expression using a flow cytometer in order to enrich for GFP/Editing agent expressing cells [Chiang, T.W., et al., CRISPR-Cas9(D10A) nickase-based genotypic and phenotypic screening to enhance genome editing. Sci Rep, 2016. 6: p. 24356]. This enrichment step allows bypassing antibiotic selection and collecting only cells transiently expressing the fluorescent protein, Cas9 and the sgRNA. These cells can be further tested for editing of the target gene by non-homologues end joining (NHEJ) and loss of the corresponding gene expression.

### Colony formation

The fluorescent protein positive cells were partly sampled and used for DNA extraction and genome editing (GE) testing and partly plated at high dilution in liquid medium to allow colony formation for 28-35 days. Colonies were picked, grown and split into two aliquots. One aliquot was used for DNA extraction and genome editing (GE) testing and CRISPR DNA-free testing (see below), while the others were kept in culture until their status was verified. Only the ones clearly showing to be GE and CRISPR DNA-free were selected forward.

After 20 days in the dark (from splitting for GE analysis, i.e., 60 days, hence 80 days in total), the colonies were transferred to the same medium but with reduced glucose (0.46 M) and 0.4 % agarose and incubated at a low light intensity. After six weeks agarose was cut into slices and placed on protoplast culture medium with 0.31 M glucose and 0.2 % gelrite. After one month, protocolonies (or calli) were subcultured into regeneration media (half strength MS + B5 vitamins, 20 g/l sucrose). Regenerated plantlets were placed on solidified media (0.8 % agar) at a low light intensity at 28 °C. After 2 months' plantlets were transferred to soil and placed in a glasshouse at 80-100 % humidity.

### Screen for gene modification and absence of CRISPR system DNA

From each colony DNA was extracted from an aliquot of GFP-sorted protoplasts (optional step) and from protoplasts-derived colonies and a PCR reaction was performed with primers flanking the targeted gene. Measures are taken to sample the colony as positive colonies will be used to regenerate the plant. A control reaction from protoplasts subjected to the same method but without Cas9-sgRNA is included and considered as wild type (WT). The PCR products were then separated on an agarose gel to detect any changes in the product size compared to the WT. The PCR reaction products that vary from the WT products were cloned into pBLUNT or PCR-TOPO (Invitrogen). Alternatively, sequencing was used to verify the editing event. The resulting colonies were picked, plasmids were isolated and sequenced to determine the nature of the mutations. Clones (colonies or calli) harboring mutations that were predicted to result in domain-alteration or complete loss of the corresponding protein were chosen for whole genome sequencing in order to validate that they were free from the CRISPR system DNA/RNA and to detect the mutations at the genomic DNA level.

Positive clones exhibiting the desired GE were first tested for GFP expression via microscopy analysis (compared to WT). Next, GFP-negative plants were tested for the presence of the Cas9 cassette by PCR using primers specific (or next generation sequencing, NGS) for the Cas9 sequence or any other sequence of the expression cassette. Other regions of the construct can also be tested to ensure that nothing of the original construct is in the genome.

### Plant regeneration

Ethylene production: Ethylene biosynthesis can be measure in small plantlets via gas chromatography (GC) or laser-based assays (Cristescu et al., 2013, Supra).

### EXAMPLE 2

### Genome editing in ACS and ACO genes of banana and plant regeneration

**Table 1. List of primers**

| **ID** | **Sequence/SEQ ID NO:** |
|---|---|
| 42 | Atgaggatctacggcgaggagcac/55 |
| 44 | Atggggctccacgttgatgaacac/56 |
| 46 | Atggggattcccggtgacgag/57 |
| 50 | Atggcgtgctccttcccgg/58 |
| 236 | Gtggcactgaatagggaggagttg/59 |
| 237 | Cgatcggctcatcctcaaacag/60 |
| 239 | Gagtttcgagccttcctgtaagca/61 |
| 240 | Cctgaagtctcgatcgaatctgg/62 |
| 242 | Gtggcagcgaatagggaggagctg/63 |
| 243 | Gaacggggaagttgacgacgcaattac/64 |
| 245 | Gaggcgatcgacatcctgttgcc/65 |
| 246 | Ctctatctgatctccgaggttgacc/66 |
| 249 | Ggtgcaccacgctcttgtac/67 |
| 250 | Atggattcctttccggttatcgacatg/68 |
| 251 | Ctcgagctggtcgccgag/69 |
| 277 | Accgaagcccctcttaaccc/70 |
| 278 | Gtatggctgacaccatcacc/71 |
| 321 | Ggggtcatccaaatgggacttg/72 |
| 322 | Ggctatatataagtagcaacg/73 |
| 323 | Acactccagatagaaagcac/74 |

sgRNAs and target sequences are described in Figure 26.

A robust protocol for the efficient isolation of protoplasts from *Musa acuminata* cells suspensions was followed according to Example 1 above, to subsequently transfect them with plasmids carrying the CRISPR/Cas9 machinery to target the genes of interest (endogenous ACS and ACO genes) and enrich for cells expressing a reporter using FACS sorting. To achieve this aim, the present inventors (i) generated and maintained embryogenic material; (ii) isolated protoplasts from that material; (iii) transfected with specific plasmids targeting ACS and/or ACO genes; (iv) enriched for cells expressing a fluorescent marker as a proxy for cells (e.g., mCherry) that carry the CRISPR/Cas9 complex and sgRNAs that target the gene of interest; and (v) advanced sorted protoplasts through a protoplast-regeneration pipeline to regenerate plantlets.

To test whether viable protoplasts from *Musa acuminata* plant material could be recovered, banana plant material (cell suspensions) was incubated in a digestion solution for 4-24h at room temperature with gentle shaking. After digestion, the plant material was washed, filtered and re-suspended in 2 ml of MMG buffer (0.4M mannitol, 15mM MagCl2, 4mM MES pH 5.6)). Protoplast concentration was determined and adjusted to 1 × 10⁶. Next, DNA plasmid pAC2010 (carrying mCherry as fluorescent marker) was incubated with the protoplasts derived from banana in the presence of polyethylene glycol (PEG). The expression of mCherry in the protoplasts was detected by fluorescence microscopy 3 days post transfection (Figure 3).

The next step in recovering gene-edited plants was to deliver the CRISPR/Cas9 complex and sgRNAs that target genes of interest in banana protoplasts and enrich for cells that carry such complex by fluorescence-activated cell sorting (FACS), thereby separating successfully transfected banana cells that transiently express the fluorescent protein, Cas9 and the sgRNA. Using FACS, positive mCherry expressing protoplasts were enriched and collected (Figure 4A). It was confirmed that the sorted protoplasts were still intact and indeed expressing the fluorescent marker by fluorescence microscopy (Figure 4B).

The transient nature of the transfection of the CRISPR/Cas9 complex and sgRNAs that target genes of interest in *Musa acuminata* protoplasts was next examined. Since all our plasmids consist of a fluorescent marker (e.g. dsRed, mCherry), Cas9, and sgRNAs (under a U6 promoter and targeting an endogenous gene of interest), the expression of the fluorescent marker in transfected banana protoplasts was followed over time and the number of mCherry-positive protoplasts was used as a proxy to get an indication of how long the CRISPR/Cas9 complex and sgRNAs might be expressed (Figures 5A-C). FACS was used to quantify the percentage of mCherry-positive banana protoplasts over time and set the total number of mCherry-positive banana protoplasts at 3 days post transfection (dpt) as 100 %. It was found that already at 10 dpt, mCherry-positive banana protoplasts decreased by 30 % of the initial number of mCherry-positive banana protoplasts and by 25 dpt almost 80 % of transfected banana protoplasts did not show any fluorescence (Figure 5C). mCherry expression was also monitored in non-sorted banana protoplasts by microscopy at 3 dpt (Figure 5A; Figure 6A), 6 dpt (Figure 6A) and 10 dpt (Figure 5B; Figure 6A), which confirmed that indeed mCherry expression diminishes over time. Moreover, fluorescence microscopy of sorted banana protoplasts shows the progressive reduction in number and intensity of mCherry-positive protoplasts (Figure 6B) as seen by FACS (Figure 4A). Taken all together, these results indicate that the expression of vectors carrying the CRISPR/Cas9 complex and sgRNAs is transient and no further Cas9 activity or integration in the plant genome is expected.

To reduce ethylene levels in banana plants, which may result in extended shelf-life of banana fruits, knockout of genes involved in the biosynthesis of ethylene, including the highlighted ACS and ACO (Figure 7A, 7B) was attempted. However, the banana genome contains multiple sequences that are homologous to these genes.

In order to identify the genes within the banana genome, which encode functional ACS and ACO, homologous sequences from characterized pathways in model or crop species were identified. The process involves a series of sequential steps for comparative analysis of DNA and protein sequences that aim at reconstructing the evolutionary history of genes through phylogenetic analysis, filtering candidates by validating their expression in general and target tissue, and sequencing of candidate genes to ensure appropriate sgRNA design (to avoid mismatches). This procedure allowed the selection of genes, the identification of optimized target regions for knockout (conserved and potentially catalytic domains), and the design of appropriate sgRNAs.

This pipeline is based on the assumption that homologous proteins with a common ancestor may have a similar function and by doing a phylogenetic reconstruction, gene families are established and assessed for functional diversity in the evolutionary context. This is particularly important for plant species that have undergone large-scale genome duplications and for expanded gene families. Nevertheless, paralogs within a gene family do not necessarily have the same function and part of the process is to target a selection of genes within a family either individually or as a group to also account for redundancy.

Briefly, synthesis of ethylene involves a three-step reaction: the enzyme S-adenosyl-methionine synthase (S-AdoMet) catalyzes adenosylation of methionine. Then S-AdoMet is metabolized to the first compound committed to ethylene biosynthesis 1-aminocyclopropane-1-carboxylic acid (ACC) by the enzyme ACC synthase (ACS). Finally, ACC is converted to ethylene by the enzyme ACC oxidase (ACO) (Figure 7A) (Cara and Giovannoni. 2008. Plant Science. Vol. 175. Pp. 106-113). During ripening, in climacteric fruits like banana, both ACC synthase (ACS) and ACC oxidase (ACO) are induced and contribute to the regulation of ethylene biosynthesis (Figure 7B) (Liu et al., 1999. Plant Physiology. Vol 121, pp. 1257-1265). Regulation of ethylene has been proposed as a two-system process in which system 1 is functional during normal vegetative growth and ethylene has an auto-inhibitory role and is responsible for producing basal ethylene levels that are detected in all tissues, including those of non-climacteric fruits while System 2 functions during ripening of climacteric fruits and maybe senescence (Figures 7A-B). At the transition stage, ripening regulators have been identified such as RIN, CNR etc, and also the induction of specific ACS gene (*LeACS4*) that leads to auto-catalysis of ethylene, which results in negative feedback on system 1. In addition, other ACS and ACO genes (*LeACS2,* 4 and *LeACO1,* 4) are induced and are responsible for the high ethylene production through system 2 (Figure 7A) (Cara and Giovannoni. 2008. Plant Science. Vol. 175. Pp. 106-113).

Whole-genome sequence analysis of *Musa acuminata* revealed specific ancestral whole-genome duplications (WGD) in the *Musa* lineage and their impact on gene fractionation (D'Hont et al., 2012. Nature. Vol 488; Martin et al., 2016. BMC Genomics. 17:243). Moreover, it has been reported that some banana gene families involved in ethylene biosynthesis and signaling evolved through WGD and were preferentially retained (Jourda et al., 2014. New Phytologist. Vol. 202. Pp 986-1000). Interestingly, major genes in the ethylene pathway are expanded and gene expression profiles suggested functional redundancy for several of those genes derived from WGD (Jourda et al., 2014. New Phytologist. Vol. 202. Pp 986-1000). Therefore, selection of candidate genes requires careful assessment.

The ethylene biosynthesis pathway has been well-studied in tomato and ACS and ACO genes involved in steps along system 1 and 2 have been characterized. These characterized genes were used as query sequences and are highlighted in Figure 9 and Figure 10 for ACS and ACO, respectively. Similarity searches confirmed that both the ACS and ACO families are e in banana (Figures 8, Figure 9, respectively) and several ACS and ACO gene candidates were selected for further studies. Sequencing of these candidates in distinct banana varieties allowed for specific design and selection of sgRNAs as shown in Figure 10. In addition, to get some insights into the possible roles of these genes, the publicly available expression data of ripening banana fruits was retrieved for all ACS and ACO candidate genes (ACS: Ma09_g19150; Ma04_g35640; Ma04_g31490. ACO: Ma01_g11540; Ma07_g19730) (Figure 11 and Figure 12, respectively). The RPKM data of each gene from the banana transcriptome database indicate that ACS Ma04_g35640 and ACO Ma07_g19730 are the candidates genes to target to reduce ethylene biosynthesis (Figure 11 and Figure 12, respectively). Embodiments of the invention also contemplate targeting other ACO and/or ACS genes to obtain a robust phenotype.

ACS genes (Ma09_g19150; Ma04_g35640; Ma04_g31490) were targeted with two pairs of sgRNAs as indicated in Figure 13A, Figure 14A, and Figure 15A. The sgRNAs are positioned between exon 1 and exon 3 of the candidate genes and these regions were selected because they are highly conserved among all 3 candidate genes. Similarly, ACO genes (Ma01_g11540; Ma07_g19730) were targeted with two pairs of sgRNAs as indicated in Figure 6A and Figure 17A. The sgRNAs are positioned between exon 1 and exon 4 of the two candidate genes and are specifically designed for each gene but combined in the transfection plasmid. sgRNAs were cloned into transfection plasmids which contained mCherry, Cas 9, and two sgRNAs driven by a U6 pol 3 promoter.

Next, the CRISPR/Cas9 complex and sgRNAs that target ACS and ACO candidates gene were transfected into banana protoplasts and enriched for cells that carry such complex by fluorescence-activated cell sorting (FACS). Using the mCherry marker, transfected banana cells that transiently express the fluorescent protein, Cas9 and the sgRNA were separated, sorted and collected mCherry-positive banana protoplasts at 3 days post transfection (dpt). DNA was extracted from 5000 sorted protoplasts (Qiagen Plant Dneasy extraction kit) at 6 dpt. Nested PCR was performed for increased sensitivity using primers shown in Figures 13A, 14A, 15A, 16A, 17A. Agarose gels of the amplified region for all candidates ACS and ACO genes are shown in Figures 13B, 14B, 15B, 16B, 17B. Only for ACO gene Ma01_g11540 a clear deletion is observed of around 350bp (Figure 17B).

To assess whether the sgRNAs and the CRISPR/Cas9 complex was active and induced genome-editing events in all other ACS and ACO genes, a T7E1 assay was performed. It was found that all sgRNA combinations induced genome-editing events in all ACS and ACO genes (ACS: Ma09_g19150; Ma04_g35640; Ma04_g31490. ACO: Ma01_g11540; Ma07_g19730) Figures 13C, 14C, 15C, 16C, 17C. Moreover, cloning and sequencing confirmed the T7E1 results for some of the genes and it was found that some of the sgRNAs used indeed induced indels as shown in Figures 13D, 15D, 18, 19, 20A, 20B. In conclusion, these results demonstrate that the CRISPR/Cas9 system can successfully be used to introduce precise mutations in the endogenous ACS and ACO genes and that the design and selection of sgRNAs impact the efficiency of genome-editing.

In parallel, additional sorted mCherry-positive protoplasts were advanced in the protoplasts regeneration. Briefly, sorted protoplasts were plated at high dilution in liquid medium to allow colony formation for 28-35 days. Colonies were picked, grown and split into two aliquots. One aliquot was used for DNA extraction and genome editing (GE) testing and CRISPR DNA-free testing while the others were kept in culture until their status was verified. Only the ones clearly showing to be GE and CRISPR DNA-free were selected forward.

After 20 days in the dark (from splitting for GE analysis, i.e., 60 days, hence 80 days in total), the colonies were transferred to the same medium but with reduced glucose (0.46 M) and 0.4 % agarose and incubated at a low light intensity. After six weeks agarose was cut into slices and placed on protoplast culture medium with 0.31 M glucose and 0.2 % gelrite. After one month, protocolonies (or calli) were subcultured into regeneration media (half strength MS + B5 vitamins, 20 g/l sucrose). (Figures 23A-E). Next, mature embryos were passed to germination medium (GM) containing MS salts and vitamins where the embryos begin to germinate 1-2 weeks after transfer. 3-4 weeks later, germinating embryos are ready to be transferred to proliferation medium for shoot elongation (Figure 24A-D).

In addition, banana embryogenic cell suspensions (ECS) were bombarded with the same plasmids used for transfection (pAC2007, pAC2008, pAC2010, pAC2011, and pAC2012) to extend shelf life. 3 days old ECS after bombardment the cells were moved to proliferation medium and as embryos develop from bombarded ECS, embryos were passed to embryo development medium (EDM) and maturation medium (Figure 25A-E).

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

### Aspects of the Invention

1. A banana plant comprising a genome comprising a loss of function mutation in a nucleic acid sequence encoding a component in an ethylene biosynthesis pathway of the banana.
2. A method of increasing shelf-life of banana, the method comprising:
   (a) subjecting a banana plant cell to a DNA editing agent directed at a nucleic acid sequence encoding a component in an ethylene biosynthesis pathway of the banana to result in a loss of function mutation in said nucleic acid sequence encoding said ethylene biosynthesis pathway and
   (b) regenerating a plant from said plant cell.
3. The method of aspect 2 further comprising harvesting fruit from said plant.
4. The plant or method of any one of aspects 1-2, wherein the plant is devoid of a transgene encoding the DNA editing agent.
5. The plant or method of any one of aspects 1-4, wherein said mutation is in a homozygous form.
6. The plant of aspect 1, 4 or 5 or ancestor thereof having been treated with a DNA editing agent directed to said genomic sequence encoding said component in said ethylene biosynthesis pathway.
7. The plant or method of any one of aspects 1-6, wherein said mutation is selected from the group consisting of a deletion, an insertion an insertion/deletion (Indel) and a substitution.
8. The plant or method of any one of aspects 1-6, wherein said component in said ethylene biosynthesis pathway is selected from the group consisting of 1-aminocyclopropane-1-carboxylate synthase (ACS) and ACC oxidase (ACO)
9. A nucleic acid construct comprising a nucleic acid sequence encoding a DNA editing agent directed at a nucleic acid sequence encoding a component in an ethylene biosynthesis pathway of a banana being operably linked to a plant promoter.
10. The plant, method or nucleic acid construct of any one of aspects 2-8, wherein said DNA editing agent is of a DNA editing system selected from the group consisting of selected from the group consisting of meganucleases, Zinc finger nucleases (ZFNs), transcription-activator like effector nucleases (TALENs) and CRISPR-Cas.
11. The plant, method or nucleic acid construct of any one of aspects 2-8, wherein said DNA editing agent is of a DNA editing system comprising CRISPR-Cas.
12. The plant, method or nucleic acid construct of any one of aspects 1-11, wherein said component in said ethylene biosynthesis pathway is selected from the group consisting of Ma04_g35640 (SEQ ID NO: 9) and Ma07 _g19730 (SEQ ID NO: 27).
13. The plant, method or nucleic acid construct of any one of aspects 1-11, wherein said component in said ethylene biosynthesis pathway is selected from the group consisting of Ma09_g19150 (SEQ ID NO: 13), Ma04_g35640 (SEQ ID NO: 9), Ma04_g31490 (SEQ ID NO: 8), Ma01_g11540 (SEQ ID NO: 20) and Ma07_g19730 (SEQ ID NO: 27).
14. The plant, method or nucleic acid construct of any one of aspects 1-11, wherein said component in said ethylene biosynthesis pathway is selected from the group consisting of Ma04_g35640 (SEQ ID NO: 9) and Ma07 _g19730 (SEQ ID NO: 27).
15. The plant, method or nucleic acid construct of any one of aspects 1-11, wherein said component in said ethylene biosynthesis pathway is selected from the group consisting of Ma09_g19150 (SEQ ID NO: 13), Ma04_g31490 (SEQ ID NO: 8) and Ma01_g11540 (SEQ ID NO: 20).
16. The plant, method or nucleic acid construct of any one of aspects 1-13, wherein said DNA editing agent is directed at nucleic acid coordinates which specifically target more than one nucleic acid sequence encoding said component in said ethylene biosynthesis pathway.
17. The plant, method or nucleic acid construct of any one of aspects 1-13, wherein said DNA editing agent comprises a nucleic acid sequence at least 99 % identical to a nucleic acid sequence selected from the group consisting of SEQ ID NO: 47-54.
18. The plant, method or nucleic acid construct of any one of aspects 1-13, wherein said DNA editing agent comprises a nucleic acid sequence at least 99 % identical to a nucleic acid sequence set forth in SEQ ID NO: 47.
19. The plant, method or nucleic acid construct of any one of aspects 1-13, wherein said DNA editing agent comprises a nucleic acid set forth in SEQ ID NO: 47.
20. The plant, method or nucleic acid construct of any one of aspects 1-13, wherein said DNA editing agent comprises a plurality of nucleic acid sequences set forth in SEQ ID NO: 47-54.
21. The plant, method or nucleic acid construct of any one of aspects 1-13, wherein said DNA editing agent comprises a plurality of nucleic acid sequences set forth in SEQ ID NO: 47, 49 or 50.
22. The plant, method or nucleic acid construct of any one of aspects 1-13, wherein said DNA editing agent comprises a plurality of nucleic acid sequences set forth in SEQ ID NO: 51 and 53.
23. A plant part of the plant of any one of aspects 1, 4-8, 10-11.
24. The plant part of aspect 23 being a fruit.
25. The fruit of aspect 24 being dry.
26. A method of producing banana, the method comprising:
   (a) growing the plant of any one of aspects 1, 4-8 and 10-11; and
   (b) harvesting fruit from the plant.
27. A processed banana product comprising genomic banana DNA comprising a loss of function mutation in a nucleic acid sequence encoding a component in an ethylene biosynthesis pathway of the banana.
28. The plant or method or processed products of any one of aspects 1-8 and 10-27, wherein the banana plant is non-transgenic.

## Claims

1. A method of increasing shelf-life of banana, the method comprising:
(a) subjecting a banana plant cell to a DNA editing agent directed at a nucleic acid sequence encoding a component in an ethylene biosynthesis pathway of the banana to result in a loss of function mutation in said nucleic acid sequence; and
(b) regenerating a plant from said plant cell.

2. The method of claim 1, further comprising harvesting fruit from said plant.

3. A banana plant comprising a genome comprising a loss of function mutation in a nucleic acid sequence encoding a component in an ethylene biosynthesis pathway of the banana.

4. A processed banana product comprising genomic banana DNA comprising a loss of function mutation in a nucleic acid sequence encoding a component in an ethylene biosynthesis pathway of the banana.

5. The method, banana plant, or processed banana product of any one of claims 1-4, wherein the plant or processed banana product is devoid of a transgene encoding the DNA editing agent.

6. The method, banana plant, or processed banana product of any one of claims 1-5, wherein said loss of function mutation:
is in a homozygous form; and/or
is selected from the group consisting of a deletion, an insertion, an insertion/deletion (Indel), and a substitution.

7. The method, banana plant, or processed banana product of any one of claims 1-6, wherein said component is selected from the group consisting of: 1-aminocyclopropane-1-carboxylate synthase (ACS) and ACC oxidase (ACO).

8. The method of any one of claims 1, 2, and 5-7, wherein said DNA editing agent is of a DNA editing system selected from the group consisting of meganucleases, Zinc finger nucleases (ZFNs), transcription-activator like effector nucleases (TALENs), and CRISPR-Cas;
preferably wherein said DNA editing agent is of a DNA editing system comprising CRISPR-Cas.

9. The method, banana plant, or processed banana product of any one of claims 1-8, wherein said component is selected from the group consisting of: Ma09_g19150 (SEQ ID NO: 13), Ma04_g35640 (SEQ ID NO: 9), Ma04_g31490 (SEQ ID NO: 8), Ma01_g11540 (SEQ ID NO: 20), and Ma07 _g19730 (SEQ ID NO: 27).

10. The method, banana plant, or processed banana product of claim 9, wherein for Ma09_g19150 (SEQ ID NO: 13), Ma04_g35640 (SEQ ID NO: 9), and/or Ma04_g31490 (SEQ ID NO: 8), the DNA editing agent is targeted between exon 1 and exon 3 of the nucleic acid sequence, and for Ma01_g11540 (SEQ ID NO: 20) and/or Ma07 _g19730 (SEQ ID NO: 27), the DNA editing agent is targeted between exon 1 and exon 4 of the nucleic acid sequence.

11. The method of any one of claims 1, 2, and 5-10, wherein said DNA editing agent comprises:
a nucleic acid sequence selected from the group consisting of SEQ ID NOs: 47-54.

12. The method of any one of claims 1, 2, and 5-10, wherein said DNA editing agent comprises a plurality of nucleic acid sequences as set forth in:
(i) SEQ ID NOs: 47-54;
(ii) SEQ ID NOs: 47, 49 or 50; or
(iii) SEQ ID NOs: 51 and 53.

13. A plant part of the banana plant of any one of claims 3, 5-7, 9, and 10;
preferably wherein the plant part is a fruit;
more preferably wherein the fruit is dry.

14. A method of producing banana fruit, the method comprising:
(a) growing the banana plant of any one of claims 3, 5-7, 9, and 10; and
(b) harvesting fruit from the banana plant.

15. The method, banana plant, or processed banana product of any one of claims 1-14, wherein the banana plant or processed banana product is non-transgenic.
